# EUROPÄISCHE PATENTSCHRIFT

(11) **EP 3 169 767 B1**
(45) Veröffentlichungstag und Bekanntmachung des Hinweises auf die Patenterteilung: **20.03.2019**
(21) Anmeldenummer: 15730080.7
(22) Anmeldetag: 03.06.2015
(51) Int. Cl.: C12M 1/24, C12M 1/00, A61M 39/10, B01F 5/10

(54) **HANDHABUNGSEINRICHTUNG MIT EINER VENTILLEITUNGSANORDNUNG**
HANDLING ARRANGEMENT HAVING A VALVE LINE ARRANGEMENT
SYSTÈME DE MANIPULATION ÉQUIPÉ D'UN ENSEMBLE DE CONDUITES DE VANNE

(30) Priorität: 18.07.2014 DE 102014214076
(43) Veröffentlichungstag der Anmeldung: 24.05.2017
(73) Patentinhaber: Hamilton Bonaduz AG, 7402 Bonaduz (CH)
(72) Erfinder: SEILER, Tobias, CH-7017 Flims Dorf (CH); KÜHNE, Oliver, CH-7208 Malans (CH); ETZOLD, Carsten, 8634 Hombrechtikon (CH)
(74) Vertreter: Ruttensperger Lachnit Trossin Gomoll
(86) Internationale Anmeldenummer: PCT/EP2015/062443
(87) Internationale Veröffentlichungsnummer: WO 2016/008636

(56) Entgegenhaltungen:
- WO-A1-2011/090781
- GB-A- 2 172 093
- US-A- 5 163 902
- US-A1- 2007 218 757
- US-A1- 2011 223 076

## Beschreibung

Die vorliegende Erfindung betrifft allgemein eine Handhabungseinrichtung mit einer Ventilleitungsanordnung, insbesondere für eine Zellkulturanlage, zur Einleitung eines Fluids in einen Laborbehälter, insbesondere Zellkulturbehälter, oder/und zur Ausleitung eines Fluids aus diesem und mit einer die Ventilleitungsanordnung aufnehmenden Tragstruktur, die Ventilleitungsanordnung umfassend ein Leitungsbauteil mit einer ersten Ankopplungsformation zur vorübergehenden Ankopplung eines ersten Fluidkanals, vorzugsweise eines Fluidkanals eines Laborbehälters, mit einer zweiten Ankopplungsformation zur vorübergehenden oder dauerhaften Ankopplung eines zweiten Fluidkanals, vorzugsweise eines Fluidkanals eines Fluidvorrats, und mit einer dritten Ankopplungsformation zur vorübergehenden oder dauerhaften Ankopplung eines dritten Fluidkanals, vorzugsweise eines Entsorgungskanals, welche Ankopplungsformationen jeweils von einem Fluidleitungsabschnitt durchsetzt sind, wobei in dem Leitungsbauteil eine Strömungskanalanordnung ausgebildet ist, durch welche jeder Fluidleitungsabschnitt der ersten, der zweiten und der dritten Ankopplungsformation mit wenigstens einem Fluidleitungsabschnitt einer der anderen beiden Ankopplungsformationen zum Fluidtransport verbunden oder verbindbar ist.

Im Zusammenhang mit der vorliegenden Erfindung ist in der vorliegenden Anmeldung beschrieben ein Fluidleitungselement zum Aufbau einer eine Mehrzahl gleicher Fluidleitungselemente umfassenden Fluidleitungsstrecke, das Fluidleitungselement umfassend einen Elementkörper, eine am Elementkörper vorgesehene erste Durchströmungsöffnung, eine am Elementkörper vorgesehene, von der ersten verschiedene zweite Durchströmungsöffnung und einen im Elementkörper vorgesehenen Strömungskanal, welcher die erste und die zweite Durchströmungsöffnung zur Durchströmung längs einer Kanalbahn strömungsmechanisch verbindet.

Ein solches Fluidleitungselement ist beispielsweise aus der WO 2011/090781 A1 der Millipore Corporation (US) bekannt.

Aus der US 5 163 902 ist ein Fluidleitungselement bekannt, umfassend einen Elementkörper, eine am Elementkörper vorgesehene erste Durchströmungsöffnung, eine am Elementkörper vorgesehene, von der ersten verschiedene zweite Durchströmungsöffnung und einen im Elementkörper vorgesehenen Strömungskanal, welcher die erste und die zweite Durchströmungsöffnung zur Durchströmung längs einer Kanalbahn strömungsmechanisch verbindet. Die US 5 163 902 betrifft allerdings Fluidleitungen zur Versorgung von Patienten, während die Handhabungseinrichtung der vorliegenden Erfindung der Versorgung von unterschiedlichen Laborbehältern durch ein und dieselbe Ventilleitungsanordnung dient.

Aus der US 2011/0223076 A1 ist eine Verzweigungs- und Sondenbaugruppe zur Verwendung an einem Biofermenter bekannt. Die Verzweigungsbaugruppe weist eine Kammer auf, welche mit dem Inneren des Fermenters kommuniziert und weist eine Mehrzahl von Öffnungen auf, welche mit der Kammer kommunizieren. Mit den Öffnungen sind längliche Sonden schraubverbunden, deren Endabschnitte in die Kammer reichen.

Aus der GB 2 172 093 A ist ein Ventil mit kurzen Reaktionszeiten zur Verwendung mit Fluid- oder Unterdruckleitungen bekannt. Dieses Ventil weist einen Permanentmagneten auf, um ein Ventilelement gegen den Ventilsitz zu drücken. Das Ventil ist normalerweise offen und ist so konstruiert, dass es nach einem Schließen nicht mehr von alleine erneut öffnet, unabhängig von Änderungen der Strömungsrichtung eines strömenden Fluids oder unabhängig von Druckänderungen. Die Schließkraft des Ventils wird dabei durch Magnete bereitgestellt.

Aus der US 2007/0218757 A1 ist ein Ventilverbinder für medizinische Infusionsleitungen bekannt, welcher umfasst: einen äußeren rohrartigen Körper mit einem Einlassende und einem Auslassende, einen inneren hohlen Dorn mit einem Zwischendichtelement, welches einen Kopf mit einem Schlitz und einem elastischen Hohlkörper umfasst, wobei der Hohlkörper wiederum mit fluiddichten Elementen ausgebildet ist, die in Berührkontakt mit dem hohlen Dorn und mit einem elastischen Schubmittel stehen, welches den elastischen Kopf in einen Schließzustand vorspannt.

Aus der WO 2011/090781 A1 ist ein Verteilersystem für einen Wegwerf-Zellkultur-Bioreaktor bekannt. Dieses Verteilersystem dient dazu, Sensoren, Fluidtestvorrichtungen, Leitungen und dergleichen mit einem Zellkultur-Bioreaktor steril zu verbinden. Das Webwerf-Bioreaktor-Verteilersystem umfasst einen von außen an einen Zellkulturbehälter anbringbaren Bioreaktor-Verteilerverbinderkörper, um folgende Baugruppen fluidmechanisch anzubringen: modulare Sensoranordnungen, welche physikalische Variablen und andere Parameter eines im Bioreaktor enthaltenen Mediums messen, Medien-Prüfkomponenten und andere Verbindungen, und ebenso wenigstens eine Leitung, welche den Bioreaktor-Verteilerverbinderkörper mit einer Pumpe verbindet, um Fluide zwischen dem Bioreaktor und dem Bioreaktor-Verteilerverbinderkörper umzupumpen.

Weiter ist in der vorliegenden Anmeldung beschrieben eine Fluidleitungsanordnung mit wenigstens einem derartigen Fluidleitungselement, bevorzugt mit einer Mehrzahl solcher Fluidleitungselemente.

Weiter ist im Zusammenhang mit der vorliegenden Erfindung beschrieben eine Ventilleitungsanordnung mit einer Fluidleitungsanordnung und einer Schaltanordnung zum Schalten wenigstens eines Ventils in der Fluidleitungsanordnung.

Die WO 2011/090781 A1 offenbart beispielsweise in ihrer Fig. 1 ein Fluidleitungselement, welches endseitig in den Bereichen seiner ersten und seiner zweiten Durchströmungsöffnung jeweils mit einem sterilen Verbinder versehen ist.

Nachteilig an dem bekannten Fluidleitungselement ist das Erfordernis seiner Sterilität. Weiter nachteilig ist der komplizierte Aufbau einer aus den bekannten Fluidleitungselementen gebildeten Fluidleitungsanordnung, bei welcher sich längs einer Kanalbahn unmittelbar benachbarte Fluidleitungselemente mit ihren jeweils aufeinander zu weisenden sterilen Verbindern berühren.

Es ist daher zunächst vorteilhaft, das aus dem Stand der Technik bekannte Fluidleitungselement der eingangs genannten Art im Sinne einer Abschwächung oder gar Vermeidung der oben genannten Nachteile zu verbessern und damit in der Folge auch aus derartigen Fluidleitungselementen gebildete Fluidleitungsanordnungen zu verbessern.

Eine solche Verbesserung wird erreicht durch ein eingangs genanntes Fluidleitungselement, bei welchem in einem näher an der ersten als an der zweiten Durchströmungsöffnung gelegenen ersten Bereich des Fluidleitungselements ein gesondert vom Elementkörper ausgebildeter Durchströmungskörper vorgesehen ist, welcher einen Teil des Strömungskanals bildet und aus einem Material mit niedrigerem Elastizitätsmodul als das Material des Elementkörpers gebildet ist und an seinem ins Innere des Elementkörpers weisenden Längsende eine um die Kanalbahn herum umlaufende Ventilsitzformation aufweist.

Der gesondert vom Elementkörper ausgebildete Durchströmungskörper kann zur Abdichtung der Verbindungsstelle mit einem weiteren gleichartigen Fluidleitungselement dienen. Hierzu bildet er einen Teil des Strömungskanals, ist also durch ein im Strömungskanal strömendes Medium durchströmbar. Dadurch besteht die Möglichkeit, den Durchströmungskörper ebenso wie den Durchströmungskanal des Fluidelements zu beliebigen Zeitpunkten zu sterilisieren, etwa indem ein geeignetes Reinigungs- oder/und Sterilisierungsmedium durch den Strömungskanal und damit den einen Teil desselben bildenden Durchströmungskörper hindurch geleitet wird.

Dadurch, dass der Durchströmungskörper weiter aus einem Material gebildet ist, welches einen niedrigeren Elastizitätsmodul aufweist als das Material des Elementkörpers, ist der Durchströmungskörper bei ansonsten gleicher äußerer Last stärker verformbar als der Elementkörper, was den Durchströmungskörper zur Verwendung als Dichtungselement am Übergang zwischen zwei längs der Kanalbahn unmittelbar benachbarten Fluidleitungselementen tauglich macht.

Weiter ist ein Durchströmungskörper pro Fluidleitungselement ausreichend, um eine längs von verbundenen einzelnen Kanalbahnen mehrerer Fluidleitungselemente verlaufende Fluidleitungsstrecke zu bilden, da jedes Fluidleitungselement einen näher an der ersten als an der zweiten Durchströmungsöffnung gelegenen Durchströmungskörper aufweist, welcher zur Abdichtung der Verbindung mit einem an die erste Durchströmungsöffnung anschließenden weiteren Fluidleitungselement ausreichend ist. So kann beispielsweise eine Fluidleitungsstrecke dadurch gebildet werden, dass die erste Durchströmungsöffnung eines Fluidleitungselements mit der zweiten Durchströmungsöffnung eines weiteren Fluidleitungselements verbunden wird. Das erstgenannte Fluidleitungselement liefert dabei den Durchströmungskörper, welcher zur Abdichtung der Verbindung zwischen dem Fluidleitungselement und dem weiteren Fluidleitungselement dienen kann. Das weitere Fluidleitungselement weist wiederum einen Durchströmungskörper an dessen erster Durchströmungsöffnung auf, welches wiederum mit der zweiten Durchströmungsöffnung eines noch weiteren Fluidleitungselements verbunden werden kann.

Zur funktionstüchtigen Bereitstellung einer aus einer Mehrzahl von Fluidleitungselementen gebildeten Fluidleitungsstrecke verbleibt daher lediglich, an einem endseitigen Fluidleitungselement die zweite Durchströmungsöffnung in geeigneter Weise zu verschließen, etwa durch einen Stopfen oder durch ein Fluidleitungselement mit einem Sackloch als Strömungskanal.

Durch die Ausbildung des Strömungskörpers mit einem Material mit niedrigerem Elastizitätsmodul als das Material des Elementkörpers ist es überdies möglich, das ins Innere des Elementkörpers weisende Längsende des Durchströmungskörpers als Ventilsitzformation auszubilden. Diese Ventilsitzformation läuft um die Kanalbahn herum, so dass der im Elementkörper vorgesehene Strömungskanal durch einen Ventilkörper für eine Durchströmung gesperrt werden kann, wenn der Ventilkörper auf die um die Kanalbahn herum umlaufende Ventilsitzformation aufgesetzt wird. Der Ventilkörper muss jedoch nicht von dem Fluidleitungselement umfasst sein.

Aufgrund der oben beschriebenen Materialwahl kann ein Ventilkörper durch das Material des Elementkörpers mit höherem Elastizitätsmodul in seiner Bewegung längs der Kanalbahn geführt werden, während die Ventilsitzformation am Durchströmungskörper aufgrund des dafür gewählten Materials mit niedrigerem Elastizitätsmodul durch den Ventilkörper verformbar sein kann, um eine möglichst gute Abdichtung zwischen Ventilsitzformation und Ventilkörper zu erreichen.

Zur Bildung einer Fluidleitungsstrecke aus dem oben genannten Fluidleitungselement kann es, wie oben bereits dargelegt wurde, ausreichen, lediglich im ersten Bereich, also näher an der ersten Durchströmungsöffnung als an der zweiten Durchströmungsöffnung, einen Durchströmungskörper vorzusehen. Daher kann ein wegen seines besonders einfachen Aufbaus bevorzugtes Fluidleitungselement derart ausgebildet sein, dass die zweite Durchströmungsöffnung und ein an diese unmittelbar in den Strömungskanal hinein anschließender, längs der Kanalbahn verlaufender zweiter Bereich des Fluidleitungselements, welcher näher an der zweiten als an der ersten Durchströmungsöffnung gelegen ist, frei von gesondert vom Elementkörper ausgebildeten Durchströmungskörpern ist.

Alternativ dazu kann es jedoch hilfreich sein, ein Fluidleitungselement als Verzweigungselement auszubilden, von dem ausgehend in unterschiedliche oder gar entgegengesetzte Richtungen eine Fluidleitungsstrecke weg verlaufen kann. Die Fluidleitungsstrecke kann durch eines oder eine Mehrzahl der oben genannten Fluidleitungselemente gebildet sein. Das Verzweigungselement kann dadurch gebildet sein, dass die beiden Durchströmungsöffnungen im Wesentlichen gleichartig aufgebaut sind, so dass sowohl an die erste Durchströmungsöffnung als auch an die zweite Durchströmungsöffnung jeweils ein wie oben beschrieben ausgestaltetes Fluidleitungselement angesetzt werden kann. Hierzu kann daran gedacht sein, dass auch in einem näher an der zweiten als an der ersten Durchströmungsöffnung gelegenen zweiten Bereich des Fluidleitungselements ein gesondert vom Elementkörper ausgebildeter zweiter Durchströmungskörper vorgesehen ist, welcher einen Teil des Strömungskanals bildet, aus einem Material mit niedrigerem Elastizitätsmodul als das Material des Elementkörpers gebildet ist und an seinem ins Innere des Elementkörpers weisenden Längsende eine um die Kanalbahn herum umlaufende Ventilsitzformation aufweist.

Der zweite Durchströmungskörper ist vorzugsweise aus dem gleichen Material gebildet wie der erste Durchströmungskörper. Aus Gründen einer möglichst einfachen Herstellung sind vorzugsweise der erste und der zweite Durchströmungskörper identisch aufgebaut.

Um sicherzustellen, dass der Durchströmungskörper Dichtungsaufgaben sowohl an der Verbindungsstelle zweier Fluidleitungselemente zur Verbindung von deren jeweiligen Strömungskanälen zu einer gemeinsamen Fluidleitungsstrecke, als auch im Zusammenwirken mit einem Ventilkörper erfüllen kann, kann der Durchströmungskörper oder/und der zweite Durchströmungskörper gemäß einer bevorzugten Weiterbildung der vorliegenden Erfindung einen Elastomerwerkstoff, insbesondere einen Kautschuk- oder/und Silikonwerkstoff, umfassen. Dies kann beispielsweise durch Beschichtung eines Kerns aus steiferem Material realisiert sein. Aus Gründen möglichst einfacher Herstellung ist bzw. sind der Durchströmungskörper oder/und der zweite Durchströmungskörper vorzugsweise aus einem derartigen Werkstoff gebildet. Bevorzugt ist der Werkstoff des Durchströmungskörpers oder/und des zweiten Durchströmungskörpers ungefüllt, um bei einer vorgegebenen Belastung einen möglichst hohen Verformungsgrad erzielen zu können.

Die Abdichtung einer Verbindungsstelle zwischen zwei längs der Kanalbahn unmittelbar benachbarten Fluidleitungselementen zur Verbindung von deren jeweiligen Strömungskanälen miteinander kann dadurch verbessert werden, dass der Durchströmungskörper oder/und der zweite Durchströmungskörper längs der Kanalbahn über den Elementkörper hinaus vorsteht bzw. vorstehen. Durch das Vorstehen des Durchströmungskörpers über den Elementkörper des ihn tragenden Fluidleitungselements hinaus kann dieser durch den Elementkörper des unmittelbar längs der Kanalbahn anschließenden weiteren Fluidleitungselements verformt und somit mit erhöhter Dichtungswirkung im Bereich zwischen den zwei benachbarten Fluidleitungselementen angeordnet sein. Die erwähnte Verformung kann beispielsweise zur Anlage des verformten Durchströmungskörpers an ihn umgebene Abschnitte des einen oder/und des anderen Elementkörpers der zwei längs der Kanalbahn benachbarten Fluidleitungselemente führen, vorzugsweise zu einer um die Kanalbahn herum vollständig umlaufenden Anlage. Diese Anlage kann bei entsprechender Anpresskraft aufgrund der elastischen Verformung des Durchströmungskörpers eine Fluidströmung zwischen Durchströmungskörper und Elementkörper am Durchströmungskörper vorbei unterbinden.

Der über den Elementkörper hinaus vorstehende Durchströmungskörper kann mit seinem im Elementkörper gelegenen Längsende an einem Absatz oder Radialvorsprung - bezogen auf die Kanalbahn - derart anliegen, dass eine Verlagerung des Durchströmungskörpers längs der Kanalbahn in den Elementkörper hinein durch diesen Anlageeingriff verhindert ist. Hierdurch kann zum einen die Lage des Durchströmungskörpers längs der Kanalbahn relativ zum Elementkörper eindeutig definiert werden. Zum anderen kann der Elementkörper mit dem Radialvorsprung bzw. dem Absatz eine, gegebenenfalls weitere, Anlagefläche zur Anlage des Durchströmungskörpers an dem Elementkörper des ihn tragenden Fluidleitungselements aufweisen, an welchem der Durchströmungskörper durch elastische Verformung eine Dichtungswirkung bzw. Strömungssperrwirkung erzielt.

Vorteilhafte Weiterbildungen, die den Durchströmungskörper betreffen, betreffen ohne weitere gesonderte Erwähnung auch den zweiten Durchströmungskörper.

Zur möglichst flexiblen Verwendung des Fluidleitungselements, vor allem hinsichtlich der Auswahl und Realisierung eines gewünschten Strömungsverlaufs durch eine Fluidleitungsanordnung, welche durch eine Mehrzahl von Fluidleitungselementen gebildet ist, kann vorgesehen sein, dass der Durchströmungskörper oder/und der zweite Durchströmungskörper auch an seinem zur Außenumgebung des Fluidleitungselements weisenden Längsende eine um die Kanalbahn herum umlaufende weitere Ventilsitzformation aufweist bzw. aufweisen. Somit kann der Strömungskörper an seinen beiden Längsenden jeweils als Ventilsitz dienen. Dies bedeutet nicht, dass der Durchströmungskörper tatsächlich an jedem Längsende als Ventilsitz dienen muss. Jedoch besteht dann die Möglichkeit, an jedem seiner Längsenden einen geeignet geformten Ventilkörper dichtend in Anlage zu bringen und wieder von der jeweiligen Ventilsitzformation zu entfernen.

Die Ventilsitzformation oder/und die weitere Ventilsitzformation an dem bzw. den Längsenden des Durchströmungskörpers kann als Einsenkung ausgebildet sein, in die hinein ein Ventilkörper verlagert werden kann. Dadurch besteht die Möglichkeit, eine Anlagefläche mit ausreichender Erstreckung längs der Kanalbahn zwischen Ventilkörper und Ventilsitzformation zu schaffen, so dass eine dichte Anlage des Ventilkörpers an der jeweiligen Ventilsitzformation mit guter Strömungssperrwirkung realisierbar ist.

Bevorzugt ist die Einsenkung als sich von dem jeweiligen die Ventilsitzformation aufweisenden Längsende des Durchströmungskörpers längs der Kanalbahn in Richtung zum jeweils anderen Längsende des Durchströmungskörpers hin verjüngende Wandungsgestalt ausgebildet. Hierdurch kann unabhängig von Fertigungs- und Formtoleranzen des Ventilkörpers oder/und der Wandungsgestalt eine ausreichende um die Kanalbahn kontinuierlich herum verlaufende Anlagefläche des Ventilkörpers an die Ventilsitzformation sichergestellt werden. Die sich verjüngende Wandungsgestalt ist bevorzugt eine sich kontinuierlich längs der Kanalbahn verjüngende Wandungsgestalt, also eine Wandungsgestalt ohne Sprünge, die möglicherweise sperrwirkungsreduzierend wirken können. Die Einsenkung kann somit konisch ausgeführt sein, wobei dann bevorzugt die Kanalbahn die Konusachse der konischen Einsenkung ist. Die Einsenkung kann jedoch auch als negativ-kugelkalottenförmige Wandungsgestalt ausgebildet sein, was sich besonders zur Verwendung von mit der Ventilsitzformation zusammenwirkenden kugelförmigen Ventilkörpern eignet. Jedoch erreicht auch die zuvor genannte konische Einsenkung hervorragende Strömungssperrwirkungen im Zusammenwirken mit kugelförmigen Ventilkörpern.

Zur definierten Verbindung der Strömungskanäle zweier Fluidleitungselemente kann es vorteilhaft sein, wenn das erfindungsgemäße Fluidleitungselement einen in Richtung der Kanalbahn vom übrigen Elementkörper vorstehenden, um die Kanalbahn umlaufenden Bund aufweist. Der Bund kann als polygonförmiger Bund um die Kanalbahn umlaufen oder kann als kontinuierlich gekrümmter Ringbund um die Kanalbahn umlaufen.

Der Bund kann an der ersten oder an der zweiten Durchströmungsöffnung vorgesehen sein. Bevorzugt ist jedoch der Bund so vorgesehen, dass er den Durchströmungskörper oder/und den zweiten Durchströmungskörper umgibt. Dadurch kann der Durchströmungskörper über eine größere Strecke längs der Kanalbahn von Material des Elementkörpers eingefasst und an diesem gehalten sein.

Das Fluidleitungselement kann - insbesondere dann, wenn es zwar an der ersten, nicht jedoch an der zweiten Durchströmungsöffnung einen Durchströmungskörper aufweist - an seinem die jeweils andere bundfreie Durchströmungsöffnung aufweisenden oder dieser nächstgelegenen Endbereich einen längs der Kanalbahn in Richtung in den Elementkörper hinein verlaufende, um die Kanalbahn umlaufende Ausnehmung aufweisen. Gemäß der vorstehenden Ausführungen ist die bundfreie Durchströmungsöffnung vorzugsweise die zweite Durchströmungsöffnung, die frei von einem Durchströmungskörper sein kann.

Die umlaufende Ausnehmung ist zur Einführung und wenigstens abschnittsweisen Aufnahme des vorstehenden umlaufenden Bundes, wie er oben diskutiert wurde, eines weiteren, vorzugsweise gleichartigen, Fluidleitungselements ausgebildet. Durch das Einführen des umlaufenden Bundes des weiteren Fluidelements in die zugeordnete Ausnehmung eines anderen Fluidelements lassen sich die Strömungskanäle der beiden Fluidleitungselemente auf besonders einfache Weise ohne weiteres Werkzeug kollinear verbinden.

Aus diesem Grunde ist ein Innenumfangsflächenabschnitt der Ausnehmung als Komplementärgestalt eines Außenumfangsabschnitts des vorstehenden umlaufenden Bundes ausgebildet, so dass zwei Fluidleitungselemente über Ausnehmung und Bund mit kollinearen Strömungskanälen möglichst spielfrei miteinander formschlüssig verbunden werden können.

Alternativ oder zusätzlich kann die kollineare Verbindung der Strömungskanäle zweier längs einer dann gemeinsamen Kanalbahn unmittelbar benachbarten Fluidleitungselemente auch durch Verwendung des Durchströmungskörpers als Steckverbindungselement erzielt werden. Hierzu ist es lediglich erforderlich, dass der Durchströmungskörper des erfindungsgemäßen Fluidelements längs der Kanalbahn dieses Fluidelements über dessen Elementkörper hinaus vorsteht.

Daher ist es vorteilhaft, wenn das Fluidleitungselement an seinem die zweite Durchströmungsöffnung aufweisenden oder an seinem der zweiten Durchströmungsöffnung nächstgelegenen Endbereich eine längs der Kanalbahn in Richtung in den Elementkörper hinein verlaufende, um die Kanalbahn herum umlaufende Vertiefung aufweist. Diese Vertiefung kann dann zur Einführung und wenigstens abschnittsweisen Aufnahme des über den Elementkörper des zuvor genannten Fluidleitungselements vorstehenden Durchströmungskörperabschnitts ausgebildet sein.

Vorzugsweise weist die Vertiefung des Fluidleitungselements einen Radialvorsprung oder einen Absatz auf, an dem das vom Elementkörper des weiteren Fluidelements weg weisende Längsende des von diesem abstehenden Durchströmungskörpers bei der Verbindung des Fluidleitungselements mit dem weiteren Fluidleitungselement in Anlage gelangt. Dann kann nämlich der Durchströmungskörper des weiteren Elements auch an dem Radialvorsprung des Fluidleitungselements in dichtende Anlage gelangen. Bevorzugt ist gemäß einer vorteilhaften Weiterbildung der vorliegenden Erfindung die Vertiefung derart bemessen, dass bei vollständiger Annäherung von Fluidleitungselement und weiterem Fluidleitungselement die beiden Radialvorsprünge, an denen sich jeweils ein Längsende des Durchströmungskörpers anliegend abstützt, ihr Abstand längs der gemeinsamen Kanalbahn kürzer ist, als die Länge des Durchströmungskörpers längs der Kanalbahn im entspannten Zustand. Dadurch kann der Durchströmungskörper bei Verbindung von Fluidleitungselement und weiterem Fluidleitungselement durch elastische Verformung vorgespannt werden, wodurch seine Dichtungswirkung an der Verbindungsstelle der beiden Fluidleitungselemente miteinander verbessert werden kann.

Weiterhin kann zur Erhöhung der Dichtungswirkung ein Innenumfangsflächenabschnitt der Vertiefung als Komplementärgestalt eines Außenumfangsflächenabschnitts des vorstehenden Durchströmungskörperabschnitts ausgebildet sein. In diesem Fall ist es möglich, dass nicht nur die Längsenden und damit die Stirnseiten des Durchströmungskörpers, sondern auch ein Außenumfangsflächenabschnitt desselben um die Kanalbahn umlaufend an dem Innenumfangsflächenabschnitt der Vertiefung anliegt und somit eine gute Strömungssperrwirkung bereitstellt.

Selbstverständlich sind in einem besonders bevorzugten Ausführungsbeispiel sowohl die umlaufende Ausnehmung als auch die Vertiefung vorgesehen. Hierzu kann im Einzelnen vorgesehen sein, dass die umlaufende Ausnehmung einen näher an einer Außenseite des Elementkörpers gelegenen ersten Ausnehmungsbereich aufweist, und einen längs der Kanalbahn weiter von der Außenseite des Elementkörpers in Richtung in den Elementkörper hinein entfernt gelegenen zweiten Ausnehmungsbereich aufweist, welcher eine kleinere lichte Weite aufweist als der erste Ausnehmungsbereich, wobei der erste Ausnehmungsbereich zur Einführung und wenigstens abschnittsweisen Aufnahme eines vorstehenden umlaufenden Bundes eines weiteren Fluidleitungselements ausgebildet ist, wobei hierzu bevorzugt ein Innenumfangsflächenabschnitt des ersten Ausnehmungsbereichs als Komplementärgestalt eines Außenumfangsflächenabschnitts des vorstehenden umlaufenden Bundes eines weiteren Fluidleitungselements ausgebildet ist, und wobei der zweite Ausnehmungsbereich zur Einführung und wenigstens abschnittsweisen Aufnahme eines über den Elementkörper vorstehenden Durchströmungskörperabschnitts des weiteren Fluidleitungselements ausgebildet ist, wobei hierzu bevorzugt ein Innenumfangsflächenabschnitt des zweiten Ausnehmungsbereichs als Komplementärgestalt eines Außenumfangsflächenabschnitts des vorstehenden Durchströmungskörperabschnitts ausgebildet ist. Der zweite Ausnehmungsbereich entspricht dabei funktionell und konstruktiv der oben genannten Vertiefung, so dass die für die Vertiefung beschriebenen vorteilhaften Weiterbildungen auch für den zweiten Ausnehmungsbereich gelten.

Um möglichst wenig Strömungsverluste im Fluidleitungselement zu erzeugen, ist es bevorzugt, dass die erste Durchströmungsöffnung und die zweite Durchströmungsöffnung koaxial längs einer geradlinigen Kanalbahn angeordnet sind. Alternativ kann die zwischen der ersten und der zweiten Durchströmungsöffnung verlaufende Kanalbahn auch um einen rechten Winkel abgewinkelt sein. Dann treten zwar an der Stelle der Abwinklung Strömungsverluste auf, jedoch ermöglicht eine rechtwinklig abgewinkelte Kanalbahn eine sehr gute Bauraumausnutzung zur Bildung einer Fluidleitungsstrecke durch die genannten Fluidleitungselemente. Die erste und die zweite Durchströmungsöffnung sind in Kanalbahnrichtung voneinander mit Abstand angeordnet.

In manchen Fällen kann es notwendig oder hilfreich sein, wenn ein Fluidleitungselement eine Zu- oder Ableitung eines Fluids durch einen zusätzlichen Strömungskanal in den bzw. aus dem im Fluidleitungselement bereits vorhandenen Strömungskanal ermöglicht. Hierzu kann das Fluidleitungselement gemäß einer vorteilhaften Weiterbildung der vorliegenden Erfindung derart ausgestaltet sein, dass längs der Kanalbahn zwischen der ersten und der zweiten Durchströmungsöffnung wenigstens eine weitere Durchströmungsöffnung vorgesehen ist, in welche ein Zusatz-Strömungskanal mündet oder welche von einem Zusatz-Strömungskanal durchsetzt ist, wobei der Zusatz-Strömungskanal in den zwischen erster und zweiter Durchströmungsöffnung verlaufenden Strömungskanal unter einem Winkel mündet. Vorzugsweise mündet der Zusatz-Strömungskanal aus den oben genannten Gründen guter Bauraumausnutzung unter einem rechten Winkel in den zwischen erster und zweiter Durchströmungsöffnung verlaufenden Strömungskanal. Es kann jedoch auch jeder andere Winkel gewählt sein, wenn dies für den jeweils gewünschten individuellen Strömungsverlauf vorteilhaft sein sollte.

Nachfolgend wird beschrieben eine Fluidleitungsanordnung mit wenigstens einem Fluidleitungselement, wie es oben beschrieben wurde. Dieses wenigstens eine Fluidleitungselement, welches nachfolgend als "erstes Fluidleitungselement" bezeichnet ist, kann gemäß den oben beschriebenen vorteilhaften Weiterbildungen ausgestaltet sein.

Zusätzlich kann die Fluidleitungsanordnung zur gezielten strömungsmechanischen Verbindung des Strömungskanals des Fluidleitungselements mit einem weiteren Fluidleitungselement verbunden sein, welches nachfolgend als "Fluidleitungsanschlusselement" bezeichnet ist. Dieses weist die folgenden Merkmale auf: eine Elementbasisgestalt, eine an der Elementbasisgestalt vorgesehene Durchlassöffnung und einen die Elementbasisgestalt längs einer Durchlassbahn durchsetzenden Durchströmkanal, wobei im Bereich des Durchströmkanals ein Magnet vorgesehen ist und wobei das Fluidleitungsanschlusselement eine Ventilsitzausbildung und eine Anbringungsausbildung zur Anbringung an eine Tragstruktur aufweist, insbesondere eine an einer Gegenausbildung der Tragstruktur verrastbare Anbringungsausbildung aufweist.

Durch die Ventilsitzausbildung kann der Durchströmkanal des Fluidleitungsanschlusselements mittels eines geeigneten Ventilkörpers zur Durchströmung freigegeben oder gesperrt werden. Der im Bereich des Durchströmkanals vorgesehene Magnet ermöglicht dabei die Verwendung eines ferromagnetischen Ventilkörpers, so dass ein berührungsloses Freigeben oder Sperren des Durchströmkanals möglich ist, was für hochreine Laboranordnungen vorteilhaft ist.

Mit der Anbringungsausbildung kann das Fluidleitungsanschlusselement an eine Tragstruktur angebracht werden. Dies kann in besonders vorteilhafter einfacher Weise durch Verrastung der Anbringungsausbildung an einer entsprechenden Gegenausbildung der Tragstruktur erfolgen, weshalb die Anbringungsausbildung vorzugsweise als verrastbare Anbringungsausbildung ausgestaltet ist. Dies kann konstruktiv in besonders einfacher Weise beispielsweise durch einen oder mehrere federkraftbelastete Vorsprünge realisiert sein.

Der Begriff der "Elementbasisgestalt" ist vorliegend zur sprachlichen Unterscheidung vom Elementkörper des zuvor beschriebenen Fluidleitungselements gewählt. Tatsächlich ist mit dem Begriff "Elementbasisgestalt" nichts anderes als ein Elementkörper gemeint. Die oben genannten Eigenschaften eines Elementkörpers eines Fluidleitungselements treffen daher ebenso für das nun im Zusammenhang mit der Fluidleitungsanordnung genannte Fluidleitungsanschlusselement zu. Ebenso gilt das oben zur Durchströmungsöffnung Gesagte in gleicher Weise für die Durchlassöffnung des Fluidleitungsanschlusselements und gilt das oben zur Kanalbahn Gesagte ebenso für die Durchlassbahn des Fluidleitungsanschlusselements. Schließlich gilt das oben zum Strömungskanal im Fluidleitungselement Gesagte für den Durchströmkanal des Fluidleitungsanschlusselements. Die Begriffe "Durchlassöffnung", "Durchlassbahn" und "Durchströmkanal" sind im Wesentlichen zur sprachlichen Unterscheidung von der "Durchströmungsöffnung", "Kanalbahn" und "Strömungskanal" gewählt, um deren Zuordnung zum Fluidleitungsanschlusselement auch sprachlich leicht kenntlich zu machen. Auf Wiederholungen zur Beschreibung dieser Ausbildungen am Fluidleitungsanschlusselement wird daher verzichtet. Stattdessen wurde auf die Beschreibung der korrespondierenden Ausbildungen des Fluidleitungselements verwiesen.

Die Durchlassbahn des Fluidleitungsanschlusselements soll nachfolgend ebenso wie zuvor die Kanalbahn des Fluidleitungselements als Bezugsstreckenkoordinate des Elements dienen, welche eine längs der Bahn verlaufende axiale Koordinate und eine von der Bahn weg oder zu der Bahn hin verlaufende radiale Komponente definiert.

Zur erleichterten Anbringung eines Ventilkörpers und ebenso zu dessen erleichtertem Austausch ist vorteilhafterweise am Fluidleitungsanschlusselement - bezogen auf dessen Durchlassbahn - an dessen einem Längsendbereich die Ventilsitzausbildung vorgesehen. Damit diese nicht durch die Anbringung an der oben genannten Tragstruktur gestört ist und damit außerdem die Anbringung des Fluidleitungsanschlusselements an die Tragstruktur ohne Risiko einer Beschädigung oder Verschmutzung der Ventilsitzausbildung erfolgen kann, ist vorteilhafterweise die Anbringungsausbildung an dem dem die Ventilsitzausbildung tragenden Längsendbereich jeweils entgegengesetzten Längsendbereich des Fluidleitungsanschlusselements vorgesehen.

Das Vorsehen der Ventilsitzausbildung an einem Längsendbereich des Fluidleitungsanschlusselements ermöglicht außerdem, einen an der Ventilsitzausbildung angeordneten oder einen durch den Magneten am Fluidleitungsanschlusselement gehaltenen Ventilkörper auch in einem mit dem Fluidleitungsanschlusselement strömungsmechanisch verbundenen Fluidleitungselement zu nutzen.

Um eine möglichst gute Dichtigkeit der Ventilsitzausbildung im Zusammenwirken mit einem daran sitzenden Ventilkörper gewährleisten zu können, weist die Ventilsitzausbildung vorzugsweise ein weichelastisches Sitzbauteil auf. Dieses kann durch den Ventilkörper, welcher vorzugsweise ein ferromagnetisches Material aufweist oder aus einem solchen gebildet ist, unter Einwirkung der im Zusammenspiel mit dem Magneten bewirkten Magnetkraft verformt werden, so dass sich das weichelastische Sitzbauteil schmiegend an den darauf aufsitzenden Ventilkörper anlegt.

Um eine möglichst hohe Magnetkraft bei geringem Bauvolumen des Magneten bewirken zu können, ist der Magnet bevorzugt unter Beteiligung von seltenen Erden als bei bestimmungsgemäßem Gebrauch unverformbarer Festkörper ausgebildet. Daher ist das weichelastische Sitzbauteil bevorzugt gesondert vom Magneten ausgebildet. Dies soll jedoch nicht die Verwendung eines aus einem mit ferromagnetischen Partikeln gefüllten Kunststoff als den Magneten ausschließen.

Da auch die Elementbasisgestalt aus Stabilitätsgründen in der Regel als bei bestimmungsgemäßem Gebrauch des Fluidleitungsanschlusselements, verglichen mit dem Sitzbauteil, unverformbarer Festkörper ausgebildet ist, ist das weichelastische Sitzbauteil der Ventilsitzausbildung besonders bevorzugt auch gesondert von der Elementbasisgestalt ausgebildet. Als weichelastisches Sitzbauteil kommen elastomere Sitzbauteile, beispielsweise unter Verwendung von Kautschuk oder/und Silikon in Frage.

Bevorzugt sind der Durchströmungskörper des Fluidleitungselements und das weichelastische Sitzbauteil der Ventilsitzausbildung aus demselben Material gefertigt.

In Weiterbildung des Vorsehens der Ventilsitzausbildung an einem Längsendbereich des Fluidleitungsanschlusselements kann die Ventilsitzausbildung, insbesondere das Sitzbauteil, das eine Längsende des Fluidleitungsanschlusselements bilden.

Zur bauraumsparenden Anordnung des Magnets der Elementbasisgestalt und vor allem zur möglichst gleichmäßigen Kraftausübung im Zusammenwirken mit einem Ventilkörper ist bevorzugt, dass der Magnet eine durchströmbare Durchgangsöffnung aufweist und den Durchströmkanal umgibt. Aus Gründen einfacher Fertigung und Montage ist dabei bevorzugt, dass der Magnet sogar einen Abschnitt des Durchströmungskanals bildet, also von dem den Durchströmkanal durchströmenden Fluid an wenigstens seiner zum Durchströmkanal hin weisenden Seite benetzbar ist. Zur möglichst gleichmäßigen Kraftausübung ist der Magnet vorzugsweise ringförmig ausgebildet.

Zur sicheren Anbringung des Fluidleitungsanschlusselements an der Tragstruktur kann vorgesehen sein, dass die Anbringungsausbildung einen Bund, vorzugsweise Ringbund, mit wenigstens einem bezüglich der Durchlassbahn radial federnden Rastvorsprung oder mit einem bezüglich der Durchlassbahn radial hintergreifbaren starren Rastvorsprung oder mit einer bezüglich der Durchlassbahn radial hintergreifbaren Rastausnehmung aufweist.

Die Montage des Magneten kann besonders einfach erfolgen, wenn der Magnet in eine den Strömungskanal wenigstens abschnittsweise bildende Aussparung in der Elementbasisgestalt eingesetzt ist. Dann bildet der Magnet eine Strömungsquerschnittsverkleinerung des Durchströmkanals. Eine einfache Befestigung des Magnets im Durchströmungskanal ohne Werkzeug kann in vorteilhafter Weise durch Verrastung erfolgen. Diese kann konstruktiv durch eine radial im Durchströmkanal bezüglich der Durchlassbahn vorstehende Rastnase realisiert sein. Die Rastnase kann kontinuierlich um die Durchlassbahn umlaufen. Wenn geringere Verrastungskräfte gewünscht sind, kann die Rastnase mit Unterbrechungen um die Durchlassbahn umlaufen oder nur lokal in einigen wenigen Umfangsabschnitten vorgesehen sein, die dann bevorzugt in Umfangsrichtung um die Durchlassbahn äquidistant voneinander angeordnet sind.

Der oben bereits genannte Ventilkörper kann Teil der Fluidleitungsanordnung sein. Bevorzugt ruht der Ventilkörper wenigstens in einem Betriebszustand der Fluidleitungsanordnung auf der Ventilsitzausbildung des Fluidleitungsanschlusselements. Zur Bereitstellung einer möglichst guten Dichtigkeit des auf der Ventilsitzausbildung aufsitzenden Ventilkörpers ist dieser bevorzugt als Ventilkugel ausgebildet.

Das ferromagnetische Material des Ventilkörpers kann dauermagnetisiert sein oder kann im Wesentlichen unmagnetisiert lediglich auf das Magnetfeld des Magneten im Fluidleitungsanschlusselement ansprechen.

Die Fluidleitungsanordnung kann ein zweites Fluidleitungselement aufweisen, wie es oben beschrieben wurde. Das zweite Fluidleitungselement kann weiterhin eine oder mehrere der oben genannten Weiterbildungen des Fluidleitungselements aufweisen. Dann weist das erste Fluidleitungselement bevorzugt einen Zusatz-Strömungskanal auf, an welchen bevorzugt der Durchströmkanal des Fluidleitungsanschlusselements angeschlossen ist. In diesem Falle kann der Strömungskanal des ersten Fluidleitungselements Teil einer Fluidleitungsstrecke sein, zu der auch der Strömungskanal des zweiten Fluidleitungselements gehört, wobei durch den Zusatz-Strömungskanal mittels des mit einem Ventilkörper abschließbaren Durchströmkanals des Fluidleitungsanschlusselements gezielt Fluid in die unter Beteiligung der Strömungskanäle des ersten und des zweiten Fluidleitungselements gebildete Fluidleitungsstrecke eingeleitet werden kann. Die genannten Fluidleitungselemente und das Fluidleitungsanschlusselement können daher derart miteinander gekoppelt sein, dass die zweite Durchströmungsöffnung des ersten Fluidleitungselements mit der ersten Durchströmungsöffnung des zweiten Fluidleitungselements gekoppelt ist, um die Strömungskanäle des ersten und des zweiten Fluidleitungselements in Reihe miteinander zu verbinden, wobei das Fluidleitungsanschlusselement mit den mit seinem der Ventilsitzausbildung näher gelegenem Längsende mit der weiteren Durchströmungsöffnung des ersten Fluidleitungselements gekoppelt ist, um den Durchströmkanal des Fluidleitungsanschlusselements in Reihe mit dem Zusatz-Strömungskanal des ersten Fluidleitungselements zu verbinden.

Dann, wenn das Fluidleitungsanschlusselement das oben genannte weichelastische Sitzbauteil aufweist, kann dieses nicht nur zur Abdichtung mit einem Ventilkörper sondern auch zur Abdichtung gegen das erste Fluidleitungselement verwendet werden, wenn das weichelastische Sitzbauteil einen Sitzabschnitt aufweist, der zur Anlage eines Ventilkörpers daran ausgebildet ist, und weiter einen Dichtungsabschnitt aufweist, der einerseits an der Elementbasisgestalt des Fluidleitungsanschlusselements anliegt und andererseits am Elementkörper des ersten Fluidleitungselements anliegt.

Vorzugsweise umgibt das Sitzbauteil den Durchströmkanal, um in Umfangsrichtung kontinuierlich eine Abdichtung gegenüber dem Fluidleitungselement bereitzustellen.

Die oben genannten Fluidleitungselemente (einschließlich des Fluidleitungsanschlusselements) gestatten eine sehr flexible Bildung einer Fluidleitungsanordnung mit beliebig vielen Zu- und Abflussleitungen. Beispielsweise kann auch das zweite Fluidleitungselement einen Zusatz-Strömungskanal aufweisen, welcher mit dem Durchströmkanal eines weiteren Fluidleitungsanschlusselements, wie es oben beschrieben ist, zu einem gemeinsamen Kanal verbunden sein kann. Dieses zweite Fluidleitungselement kann wiederum mit einem weiteren Fluidleitungselement, wie es oben beschrieben und bevorzugt weitergebildet ist, verbunden sein, und zwar dadurch, dass seine zweite Durchströmungsöffnung mit der ersten Durchströmungsöffnung des weiteren Fluidleitungselements gekoppelt ist. Dadurch können die Strömungskanäle des zweiten und des weiteren Fluidleitungselements in Reihe miteinander verbunden werden, so dass eine Fluidleitungsstrecke unter Beteiligung der Strömungskanäle des ersten, des zweiten und des weiteren Fluidleitungselements gebildet werden kann. Bevorzugt ist diese Fluidleitungsstrecke geradlinig. In diese können die Zusatz-Strömungskanäle der Fluidleitungselemente einmünden und so Zu- oder/und Abflussverbindungen schaffen. Das weitere Fluidleitungsanschlusselement ist dann, wie oben bereits angedeutet, mit der weiteren Durchströmungsöffnung des zweiten Fluidleitungselements gekoppelt.

Das Fluidleitungsanschlusselement oder/und das weitere Fluidleitungsanschlusselement können, wie oben bereits beschrieben, einen in einem Betriebszustand auf der jeweiligen Ventilsitzausbildung ruhenden, ferromagnetisches Material umfassenden, vorzugsweise aus ferromagnetischem Material gebildeten Ventilkörper aufweisen. Dieser ist wiederum bevorzugt eine Ventilkugel.

Der Ventilkörper kann sich dann in einem Bewegungsraum befinden, der wenigstens abschnittsweise aus Strömungskanal und Zusatz-Strömungskanal des ersten Fluidleitungselements sowie aus einem Abschnitt des Durchströmkanals des Fluidleitungsanschlusselements gebildet ist oder welcher wenigstens abschnittsweise aus Strömungskanal und Zusatz-Strömungskanal des zweiten Fluidleitungselements sowie aus einem Abschnitt des Durchströmkanals des weiteren Fluidleitungsanschlusselements gebildet ist. Die zum jeweiligen Bewegungsraum hin weisenden Längsenden der Durchströmungskörper der an der Bildung des Bewegungsraums beteiligten Fluidleitungselemente weisen vorteilhafterweise die oben genannten Ventilsitzformationen auf, so dass auch diese Längsenden des oder der Durchströmungskörper als Ventilsitze nutzbar sind. Auf diese Weise können durch einen Ventilkörper unterschiedliche Strömungswege durch die Fluidleitungsanordnung realisiert werden, insbesondere durch berührungsloses Schalten mittels magnetischer Kräfte von außerhalb der Fluidleitungsanordnung.

Nachfolgend wird beschrieben eine Ventilleitungsanordnung mit einer wie oben beschrieben ausgebildeten Fluidleitungsanordnung, welche weiter eine Schaltanordnung aufweist. Die Schaltanordnung dient dazu, den Ventilkörper in dem wenigstens abschnittsweise aus Kanälen eines Fluidleitungselements und eines mit diesem verbundenen Fluidleitungsanschlusselements zwischen unterschiedlichen Ventilkörperpositionen zu schalten. Damit der Ventilkörper berührungslos zwischen unterschiedlichen Stellungen geschaltet werden kann, ist die Schaltanordnung dazu ausgebildet, in einem Schaltbereich des Bewegungsraums, welcher Schaltbereich näher an der ihm zugeordneten Ventilsitzformation des Durchströmungskörpers des ersten Fluidleitungselements als an der Ventilsitzausbildung des Fluidleitungsanschlusselements gelegen ist, oder/und in einem Schaltbereich des Bewegungsraums, welcher Schaltbereich näher an einer ihm zugeordneten weiteren Ventilsitzformation des Durchströmungskörpers des zweiten Fluidleitungselements als an der Ventilsitzausbildung des Fluidleitungsanschlusselements gelegen ist, ein bezüglich der lokal in dem Schaltbereich wirkenden Magnetfeldstärke veränderliches Magnetfeld bereitzustellen, um durch Veränderung der Magnetfeldstärke das Ventilelement von der Anlage an der Ventilsitzausbildung zur Anlage an der dem jeweiligen Schaltbereich zugeordneten Ventilsitzformation zu verlagern.

Die Zuordnung einer Ventilsitzformation zu einem Schaltbereich erfolgt durch die räumliche Nähe des Schaltbereichs zu der Ventilsitzformation. Der Schaltbereich, in welchem das Magnetfeld mit veränderlicher Magnetfeldstärke bereitgestellt werden kann, kann durch das das Magnetfeld bereitstellende Mittel identifiziert werden. Dieses kann beispielsweise ein Elektromagnet sein, welcher zur Veränderung der Magnetfeldstärke des von ihm ausgehenden Magnetfelds abhängig von gewünschten Schaltzuständen unterschiedlich stark bestromt werden kann. Dies schließt einen Betriebszustand mit unbestromtem Elektromagneten ein. In diesem Falle wird der Elektromagnet näher bei der ihm zugeordneten Ventilsitzformation gelegen sein als bei der ihm nicht zugeordneten Ventilsitzformation desselben Bewegungsraums.

Jedoch ist die Verwendung von Elektromagneten als ein veränderliches Magnetfeld bereitstellendes Mittel nicht bevorzugt, da die unterschiedlich starken Bestromungszustände des Elektromagneten zu einer Wärmeentwicklung im Umfeld des Elektromagneten führen können. Hierdurch kann Wärmeenergie ausgehend vom Elektromagneten auf ein in den Kanälen der Fluidleitungsanordnung strömendes Fluid übertragen werden, was zu einer unerwünschten Erwärmung des Fluids führen kann. Mitunter werden nämlich durch die Kanäle der Fluidleitungsanordnung thermisch sensitive Fluide geleitet, welche eine Nennbetriebstemperatur mit einer Toleranz von nur ±0,5 K aufweisen müssen.

Daher ist es zur Vermeidung einer unerwünschten Erwärmung des Fluids durch die Verwendung von Elektromagneten bevorzugt, dass die Schaltanordnung wenigstens einen längs einer Schaltbewegungsbahn an das erste Fluidleitungselement, insbesondere an eine Ventilsitzformation desselben, annäherbaren und von diesem entfernbaren Permanentmagneten umfasst.

Durch die Annäherung und Wiederentfernung des Permanentmagneten zu der zugeordneten Ventilsitzformation zu bzw. von dieser weg kann ebenfalls ein lokal im Schaltbereich veränderliches Magnetfeld bereitgestellt werden.

Die Schaltbewegungsbahn ist vorzugsweise orthogonal zur Kanalbahn an der dem Permanentmagneten zugeordneten Ventilsitzformation. Ein korrekter Sitz des Ventilkörpers auf der einem Magneten zur Erzeugung eines veränderlichen Magnetfelds zugeordneten Ventilsitzformation kann trotz der möglicherweise asymmetrischen Anordnung des Magneten bezüglich des die Ventilsitzformation durchsetzenden Kanalbahnabschnitts durch entsprechende Dimensionierung des Strömungskanals erreicht werden. Dann, wenn der Strömungskanal wenigstens in der Nähe der Ventilsitzformation eine zur Kanalbahn orthogonale lichte Weite aufweist, welche nur unwesentlich größer ist als die in gleicher Richtung zu messende Abmessung des Ventilkörpers, kann der Ventilkörper ohne übermäßige Reibungsverluste durch das veränderliche Magnetfeld des der Ventilsitzformation zugeordneten Magneten zu der Ventilsitzformation hin bewegt werden, ohne von seiner bevorzugt relativ zur Kanalbahn zentralen Position übermäßig abzuweichen. Auch aus diesem Grunde ist die Verwendung einer Ventilkugel als Ventilkörper bevorzugt, denn diese hat unabhängig von ihrer lokalen Orientierung stets allseits die gleichen Abmessungen. Als Strömungskanal kann dann ein zylindrischer Strömungskanal verwendet werden.

Eine sichere Anlage des Ventilkörpers an der dem Permanentmagneten zugeordneten Ventilsitzformation kann dadurch erreicht werden, dass die geradlinig in den Elementkörper des ersten Fluidleitungselements hinein verlängert gedachte Schaltbewegungsbahn entweder den dem Schaltbereich zugeordneten Durchströmungskörper durchsetzt, oder - bei Betrachtung des an der Ventilsitzformation anliegenden Ventilkörpers - den Bewegungsraum zwischen der dem Schaltbereich zugeordneten Ventilsitzformation und dem Ventilkörperabschnitt mit der größten zur Kanalbahn orthogonalen Abmessung durchsetzt. In diesem Falle verbleibt selbst bei orthogonal zur Kanalbahn verlaufender Schaltbewegungsbahn stets eine ausgehend vom Permanentmagneten auf den Ventilkörper einwirkende Magnetkraft, welche eine längs der Kanalbahn zur Ventilsitzformation hin wirkende Kraftkomponente besitzt. Somit wird der Ventilkörper bei angenähertem Permanentmagneten stets mit einer ausreichenden Kraft axial in Richtung zur Ventilsitzformation hin beaufschlagt.

Der Permanentmagnet kann in beliebiger Weise zur Annäherung an die Kanalbahn und zur Entfernung von dieser angetrieben sein. Denkbar sind dabei Spindeltriebe oder ähnliche elektromechanische Antriebe. Jedoch sind derartige elektromechanische Antriebe möglicherweise nicht geeignet, um besonders kurze Schaltzeiten zu erzeugen. Außerdem können von diesen selbst wiederum Magnetfelder ausgehen. Schließlich können auch diese Antriebe unerwünschterweise als Wärmequelle wirken.

Bevorzugt ist daher der annäherbare und wieder entfernbare Permanentmagnet durch eine Kolben-Zylinder-Anordnung angetrieben, wobei der Permanentmagnet bei tragstrukturfestem Zylinder mit der Kolbenstange oder bei tragstrukturfester Kolbenstange mit dem Zylinder zur gemeinsamen Bewegung gekoppelt sein kann. Als Arbeitsfluid der Kolben-Zylinder-Anordnung kommen Flüssigkeiten, wie etwa Hydrauliköl, oder ein Gas, insbesondere Luft, in Frage. Bevorzugt ist der Schaltbewegungsantrieb des Permanentmagneten pneumatisch. Die Kolben-Zylinder-Anordnung kann zur Rückstellung gegen die elastische Kraft einer Rückstellfeder arbeiten, welche den Permanentmagneten in eine Betriebsstellung vorspannt. Dann ist für die Dauer einer an der Fluidleitungsanordnung bewirkten Ventilschaltstellung der Druck in der Kolben-Zylinder-Anordnung gegen die Wirkung der Rückstellfeder aufrecht zu erhalten. Alternativ kann eine Kolben-Zylinder-Anordnung mit doppelt wirkendem Zylinder verwendet werden. Dann kann ein Druckstoß zur Erzeugung einer gewünschten Schaltbewegung des wenigstens einen Permanentmagneten ausreichen.

Dann, wenn der dem Schaltbereich zugeordnete Durchströmungskörper sowohl eine Ventilsitzformation als auch eine weitere Ventilsitzformation aufweist, durchsetzt die verlängert gedachte Schaltbewegungsbahn den Durchsetzungskörper oder den Bewegungsraum näher bei der dem Schaltbereich zugeordneten Ventilsitzformation als bei der jeweils anderen Ventilsitzformation. Hierdurch kann vermieden werden, dass unbeabsichtigt der falsche Ventilkörper an eine Ventilsitzformation eines Durchströmungskörpers zur Anlage gebracht wird.

Wie oben dargelegt wurde, kann die Fluidleitungsanordnung mehrere Bewegungsräume mit je einem Ventilkörper umfassen. Beispielsweise kann für jedes Fluidleitungsanschlusselement ein eigener Bewegungsraum mit einem eigenen Ventilkörper gebildet sein. Dann ist zur gezielten Schaltung der unterschiedlichen Ventilkörper vorzugsweise für jeden Bewegungsraum wenigstens ein Permanentmagnet vorgesehen, der jeweils an jenes Fluidleitungselement annäherbar und von diesem entfernbar ist, dessen Strömungskanal und Zusatz-Strömungskanal zur Bildung des jeweiligen Bewegungsraums beitragen.

Weist ein Bewegungsraum mehrere Ventilsitzformationen auf, beispielsweise durch eine Ventilsitzformation des Durchströmungskörpers des ersten Fluidleitungselements und durch eine weitere Ventilsitzformation des Durchströmungskörpers des mit dem ersten Fluidleitungselement gekoppelten zweiten Fluidleitungselements, dann kann für jede zu dem Bewegungsraum hin weisende und diesem zugeordnete Ventilsitzformation ein an das Fluidleitungselement annäherbarer und von diesem entfernbarer Permanentmagnet vorgesehen sein. Vorzugsweise ist dann jeder Ventilsitzformation ein annäherbarer und entfernbarer Permanentmagnet zugeordnet.

Die vorliegende Erfindung betrifft eine Handhabungseinrichtung mit einer, bevorzugt wie oben beschrieben ausgestalteten, Ventilleitungsanordnung. Die Handhabungseinrichtung umfasst weiter eine die Ventilleitungsanordnung aufnehmende Tragstruktur, eine Mehrzahl von zu der Ventilleitungsanordnung hin oder/und von dieser weg führenden Versorgungsleitungen, wobei wenigstens ein Teil der Versorgungsleitungen, vorzugsweise alle Versorgungsleitungen, jeweils einen Leitungsabschnitt aufweist bzw. aufweisen, welcher durch einen Durchströmkanal eines Fluidleitungsanschlusselements gebildet ist, und eine Anschluss-Durchströmungsöffnung, welche zum lösbaren, vorübergehenden Anschluss eines Laborbehälters oder einer zu einem Laborbehälter führenden Fluidleitung ausgebildet ist, wobei die Mehrzahl von Fluidleitungsanschlusselementen mittels einer Mehrzahl von Fluidleitungselementen mit der Anschluss-Durchströmungsöffnung strömungsmechanisch verbunden oder durch die Schaltanordnung strömungsmechanisch verbindbar ist.

Diese Handhabungseinrichtung löst die objektive technische Aufgabe, ein und dieselbe Ventilleitungsanordnung mit unterschiedlichen Laborbehältern, wie etwa Zellkulturbehältern, fluidübertragend zu koppeln. Somit kann eine Mehrzahl von Laborbehältern durch eine einzige Handhabungseinrichtung hinsichtlich Fluideinleitung und Fluidausleitung bearbeitet werden.

Vorzugsweise ist jeder Fluidleitungsabschnitt der ersten, der zweiten und der dritten Ankopplungsformation mit jedem Fluidleitungsabschnitt der anderen beiden Ankopplungsformationen zum Fluidtransport verbunden oder verbindbar.

Als Leitungsbauteil gemäß der vorliegenden Erfindung ist ein körperlich ausgebildetes Bauteil zu verstehen. Dieses kann beispielsweise als eine vorangehend beschriebene Fluidleitungsanordnung mit wenigstens einem Fluidleitungselement und einer Mehrzahl von Fluidleitungsanschlusselementen ausgebildet sein. Bei einem derartigen Leitungsbauteil kann eine erste Ankopplungsformation an einem Fluidleitungselement bereitgestellt sein, während zwei Fluidleitungsanschlusselemente zumindest Teil einer zweiten bzw. dritten Ankopplungsformation sein können. Es sei jedoch ausdrücklich darauf hingewiesen, dass das Leitungsbauteil nicht auf die zuvor beschriebene Fluidleitungsanordnung beschränkt ist. Das Leitungsbauteil kann überdies aus einer Mehrzahl von Teilbauteilen gebildet sein, wenn die Komplexität der Strömungskanalanordnung dies erfordert.

Die Strömungskanalanordnung bezeichnet im Gegensatz zum Leitungsbauteil kein körperlich ausgebildetes Bauteil, sondern lediglich einen Kanalverlauf zwischen den drei vorangehend definierten Ankopplungsformationen. Folglich existieren hinsichtlich ihrer Ausgestaltung keine grundsätzlichen Einschränkungen. Denkbar ist beispielsweise eine durch eine Mehrzahl von flexiblen Schläuchen oder aus zusammengefügten Halb- oder Teilschalen gebildete Strömungskanalanordnung. Bei einem als Fluidleitungsanordnung ausgebildeten Leitungsbauteil bilden der zuvor beschriebene Strömungskanal und der Zusatz-Strömungskanal einen Teil der Strömungskanalanordnung, welche durch wenigstens ein Fluidleitungselement als körperliches Bauteil definiert ist.

In Weiterbildung der Erfindung kann vorgesehen sein, dass die Ventilleitungsanordnung einen relativ zum Leitungsbauteil beweglichen Fluidkanalträger aufweist, welcher wenigstens zwei gesondert voneinander ausgebildete und mit unterschiedlichen Fluidkanälen zum Fluidtransport verbundene, jeweils von einem Fluidkanalabschnitt durchsetzte Fluidkanalträger-Anschlussformationen aufweist, und wobei wenigstens eine der drei Ankopplungsformationen als Wechsel-Ankopplungsformation zur vorübergehenden Herstellung einer gemeinsamen Fluidleitungsstrecke mit einer durch Relativbewegung von Leitungsbauteil und Fluidkanalträger auswählbaren Fluidkanalträger-Anschlussformation ausgebildet ist.

Bei dieser Ausgestaltung weist der Fluidkanalträger wenigstens zwei gesondert voneinander ausgebildete und mit unterschiedlichen Fluidkanälen zum Fluidtransport verbundene Fluidkanalträger-Anschlussformationen auf, von welchen jede zur Herstellung einer gemeinsamen Fluidleitungsstrecke mit der Wechsel-Ankopplungsformation des Leitungsbauteils vorübergehend koppelbar ist. Diejenige Fluidkanalträger-Anschlussformation, welche mit der Wechsel-Ankopplungsformation des Leitungsbauteils eine gemeinsame Fluidleitungsstrecke bilden soll, kann in einfacher Weise durch Relativbewegung von Fluidkanalträger und Leitungsteil ausgewählt werden. Somit können hier in vorteilhafter Weise eine größere Anzahl an zu der Ventilleitungsanordnung hin oder/und von dieser weg führenden Fluidkanälen dauerhaft mit der Ventilleitungsanordnung verbunden sein als Ankopplungsformationen am Leitungsbauteil vorgesehen sind. Somit können Ankopplungsformationen am Leitungsbauteil eingespart werden, was wiederum zu einer Bauraumeinsparung führt.

Die unterschiedlichen am Leitungsbauteil vorgesehenen Ankopplungsformationen und die diese verbindende Strömungskanalanordnung sollten zur Erhöhung der Betriebshygiene für unterschiedliche Fluide unterschiedliche Fluidleitungsabschnitte bereitstellen können. Dies kann in vorteilhafter Weise dadurch geschehen, dass eine Mehrzahl von Ankopplungsformationen, vorzugsweise alle Ankopplungsformationen mit Ausnahme der Ankopplungsformation zur vorübergehenden Ankopplung eines Fluidkanals eines Laborbehälters, einen Ventilsitz und einen Ventilkörper aufweist bzw. aufweisen, wobei der Ventilkörper in einem Betriebszustand mit für eine Fluiddurchleitung gesperrtem Fluidleitungsabschnitt einer Ankopplungsformation auf dem Ventilsitz ruht.

In einer besonders bevorzugten Ausführungsform kann hierbei weiter vorgesehen sein, dass in der Strömungskanalanordnung abseits der Ankopplungsformationen ein Ventilsitz und ein Ventilkörper zur strömungsmechanischen Trennung verschiedener Bereiche der Strömungskanalanordnung vorgesehen sind, wobei in einem Betriebszustand mit voneinander strömungsmechanisch getrennten Bereichen der Strömungskanalanordnung der Ventilkörper auf dem Ventilsitz ruht.

Diese Weiterbildung lässt sich baulich vorzugsweise dadurch realisieren, dass der Ventilkörper wenigstens teilweise, vorzugsweise vollständig, aus ferromagnetischem Material gebildet ist und dass der Ventilsitz einen Magneten aufweist, welcher den Ventilkörper magnetisch in eine Schließstellung vorspannt, in der der Ventilkörper auf dem Ventilsitz ruht. Für eine möglichst homogene Kraftverteilung bei der Vorspannung des Ventilkörpers zum Ventilsitz hin kann es vorteilhaft sein, einen ringförmigen Magneten zu verwenden. Bevorzugt ist der den Ventilkörper in die Schließstellung vorspannende Magnet von Fluid durchströmbar, so dass er mit geringem Bauraumaufwand in oder nahe der den Ventilsitz aufweisenden Ankopplungsformation vorgesehen sein kann.

Eine besonders vorteilhafte Möglichkeit, den Ventilkörper berührungslos und damit hochhygienisch zwischen zwei Stellungen zu schalten, von welchen der Ventilkörper in einer einen Fluiddurchgang durch den Ventilsitz sperrt und in einer anderen den Fluiddurchgang durch den Ventilsitz gestattet, kann dadurch realisiert sein, dass der Ventilkörper ferromagnetisches Material aufweist, insbesondere aus ferromagnetischem Material hergestellt ist, und dass die Ventilleitungsanordnung eine Schaltanordnung mit lokal an den Ankopplungsformationen veränderbarer magnetischer Feldstärke aufweist, mit welcher der Ventilkörper von dem Ventilsitz lösbar ist.

Ist ein Ventilkörper zur Anlage an mehreren Ventilsitzen vorgesehen, kann es ausreichen, dass nur einer dieser Ventilsitze einen Magneten aufweist. In Anlage an den anderen Ventilsitzen kann der Ventilkörper vorübergehend durch die Schaltanordnung gehalten werden.

Jede der vorangehend beschriebenen Handhabungseinrichtungen kann zum Ankoppeln des Laborbehälters an die Ventilleitungsanordnung ein Bewegungsteil aufweisen, wie etwa einen Schlitten oder einen Wagen. Das Bewegungsteil ist zur vorübergehenden Aufnahme eines Laborbehälters ausgebildet und ist längs einer Anschlussbahn an die Ventilleitungsanordnung annäherbar und von dieser entfernbar. Durch das Bewegungsteil kann der daran aufgenommene Laborbehälter sicher geführt und somit mit großer Betriebssicherheit an die Ventilleitungsanordnung, insbesondere an die Anschluss-Durchströmungsöffnung, angekoppelt und von dieser wieder abgekoppelt werden.

Insbesondere dann, wenn Zellkulturbehälter als Laborbehälter verwendet werden, kann gewünscht sein, dass Innenflächen des Laborbehälters mit einer in den Laborbehälter eingeleiteten Flüssigkeit benetzt werden. Hierzu kann vorgesehen sein, dass das Bewegungsteil und die Ventilleitungsanordnung wenigstens dann, wenn sich das Bewegungsteil in einer an die Ventilleitungsanordnung angenäherten Stellung befindet, um eine gemeinsame, vorzugsweise orthogonal zur Anschlussbahn verlaufende, Schwenkachse schwenkbar an einem Tragrahmen der Handhabungseinrichtung vorgesehen sind. Somit kann über die Ventilleitungsanordnung eine Flüssigkeit, beispielsweise eine Nährflüssigkeit für Zellkulturen, in den Zellkulturbehälter eingeleitet werden, wobei der Laborbehälter unmittelbar nach Einleiten der Flüssigkeit noch im Zustand einer Ankopplung an die Ventilleitungsanordnung um die Schwenkachse verschwenkt werden kann, um eine gezielte Benetzung von Innenflächen des Laborbehälters mit der eingeleiteten Flüssigkeit zu erreichen.

Als Schwenkantrieb kann ein elektromotorischer Antrieb vorgesehen sein, welcher dazu eingerichtet ist, eine Antriebskraft auf eine mit dem Tragrahmen zur gemeinsamen Schwenkbewegung gekoppelte Schwenkwelle zu übertragen. Ein einfacher und kompakter Aufbau kann dabei dadurch bereitgestellt werden, dass der Schwenkantrieb einen um eine mit der Schwenkwelle drehfest verbundene Riemenscheibe verlaufenden Riemen oder/und ein Zahnradgetriebe umfasst. Alternativ ist es auch möglich, dass der Schwenkantrieb unmittelbar mit der Schwenkwelle gekoppelt ist bzw. die Schwenkwelle ist. Darüber hinaus sind auch Ausgestaltungen denkbar, bei welchen keine Schwenkwelle vorgesehen ist, sondern beispielsweise lediglich ein drehfest mit dem Bewegungsteil verbundenes Zahnrad, auf welches der Schwenkantrieb eine Antriebskraft übertragen kann.

Die vorliegende Erfindung wird nachfolgend anhand der beiliegenden Zeichnungen näher beschrieben werden. Es stellt dar:
- Figur 1: eine perspektivische Ansicht eines Teils einer erfindungsgemäßen Handhabungseinrichtung,
- Figur 2: den Teil der Handhabungseinrichtung von Figur 1 in einem Betriebszustand mit an eine Fluidleitungsanordnung angekoppeltem Laborbehälter,
- Figur 3: eine erfindungsgemäße Handhabungseinrichtung mit dem in den Figuren 1 und 2 bereits dargestellten Einrichtungsteil,
- Figur 4: die Handhabungseinrichtung von Figur 3 in einem weiteren Betriebszustand,
- Figur 5a: eine Schnittansicht durch die Ventilleitungsanordnung von Figur 8 entlang der Schnittebene Va-Va in Fig. 8, die Ventilleitungsanordnung umfasst die Fluidleitungsanordnung der Handhabungseinrichtung der Figuren 1 bis 4,
- Figur 5b: eine vergrößerte Darstellung eines Ausschnitts der Schnittansicht von Figur 5a, jedoch mit Ventilkörpern an den jeweiligen Ventilsitzausbildungen der Fluidleitungsanschlusselemente anliegend, entsprechend dem Betriebszustand von Fig. 7,
- Figur 6: eine perspektivische Ansicht der Ventilleitungsanordnung der Handhabungseinrichtung von Fig. 2 (mit angekoppeltem Laborbehälter),
- Figur 7: eine Längsschnittansicht durch die Ventilleitungsanordnung von Figur 6 mit Blickrichtung zu dem Laborbehälter hin,
- Figur 8: eine Schnittansicht der Ventilleitungsanordnung von Fig. 5a entlang der Schnittebene VIII-VIII von Figur 5a; die Ansicht von Fig. 8 entspricht auch der Längsschnittansicht von Figur 7, jedoch mit einem anderen Betriebszustand der Ventilleitungsanordnung,
- Figur 9: eine perspektivische Ansicht eines Teils einer zweiten Ausführungsform einer erfindungsgemäßen Handhabungseinrichtung und
- Figur 10: eine Längsschnittansicht durch den in Figur 9 gezeigten Teil der Handhabungseinrichtung gemäß der zweiten Ausführungsform.

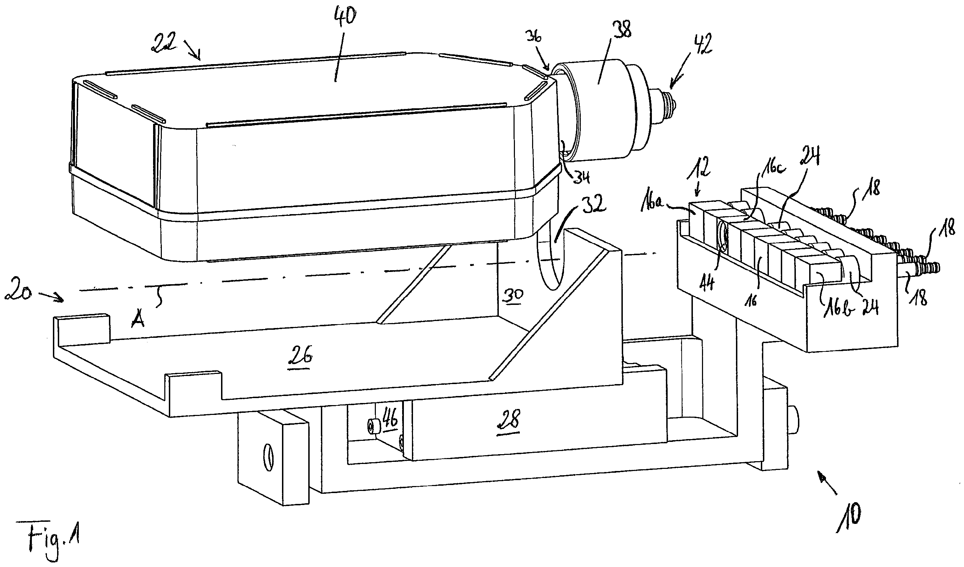

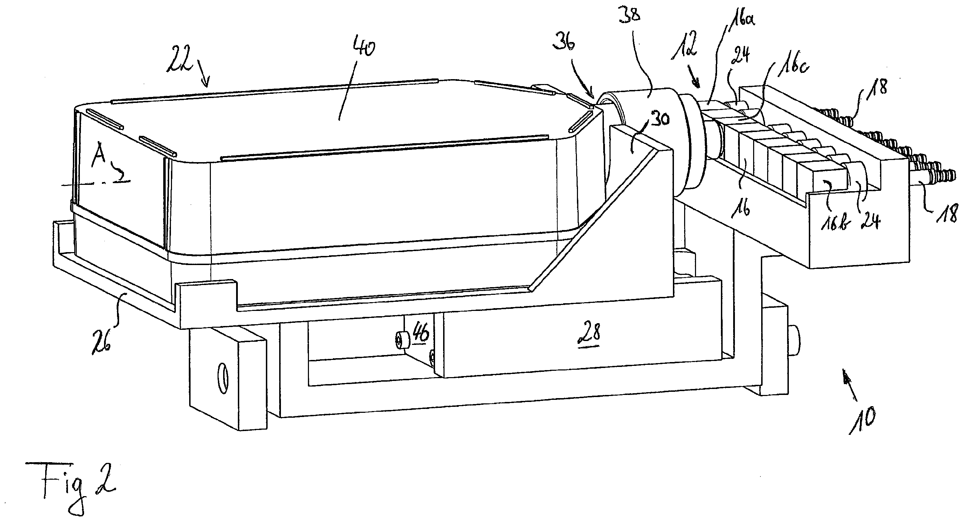

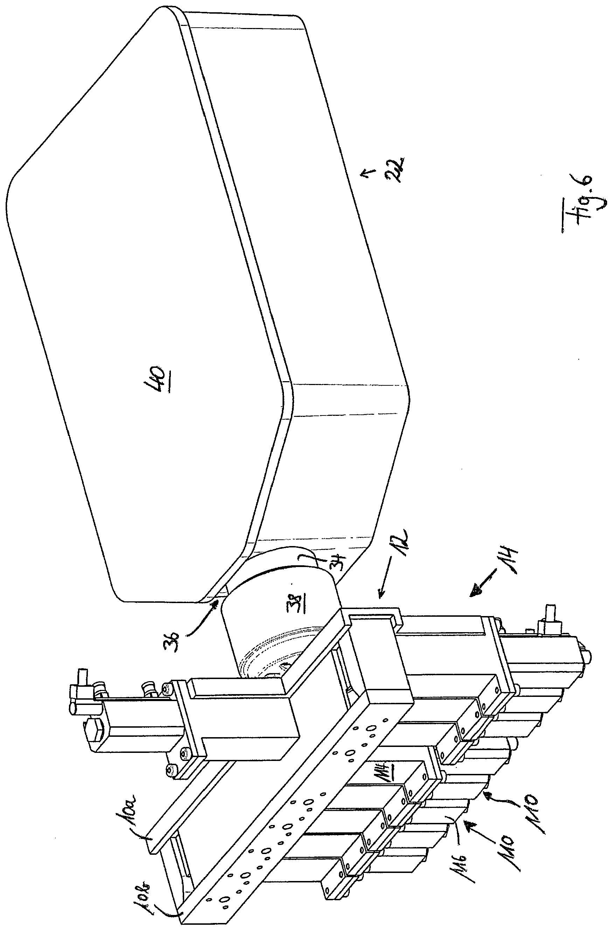

In den Figuren 1 und 2 ist eine Tragstruktur allgemein mit 10 bezeichnet. In der Tragstruktur ist eine Ventilleitungsanordnung aufgenommen, von der in den Figuren 1 und 2 lediglich die Fluidleitungsanordnung 12 dargestellt ist. Eine ebenfalls zur Ventilleitungsanordnung gehörende Schaltanordnung 14 ist in den Figuren 6 bis 8 dargestellt.

Die Fluidleitungsanordnung 12 weist eine Mehrzahl von strömungsmechanisch miteinander verbundenen Fluidleitungselementen 16 auf. Jene Fluidleitungselemente 16, welche in der dargestellten Fluidleitungsanordnung keine endseitigen Fluidleitungselemente 16a oder 16b sind, können jeweils einen in den Figuren 1 und 2 nicht dargestellten Strömungskanal und einen davon in einem rechten Winkel abzweigenden Zusatz-Kanal aufweisen. An einer weiteren Durchströmungsöffnung der Fluidleitungselemente 16, mit welchen die jeweiligen Zusatz-Strömungskanäle der einzelnen Fluidleitungselemente 16 zur Außenumgebung der Fluidleitungselemente 16 hin öffnen, können Anschlussstutzen 18 vorgesehen sein, an welche Fluidleitungen anschließbar sind, die das jeweilige Fluidleitungselement 16 und damit die Fluidleitungsanordnung 12 mit Fluidvorräten oder Abflussbehältern ("waste") verbinden können.

Ein Fluidleitungselement 16c ist derart orientiert in der Fluidleitungsanordnung 12 angeordnet, dass seine weitere Durchströmungsöffnung zu einer Laborbehälteraufnahme 20 öffnet, an welcher ein Laborbehälter 22 aufnehmbar ist.

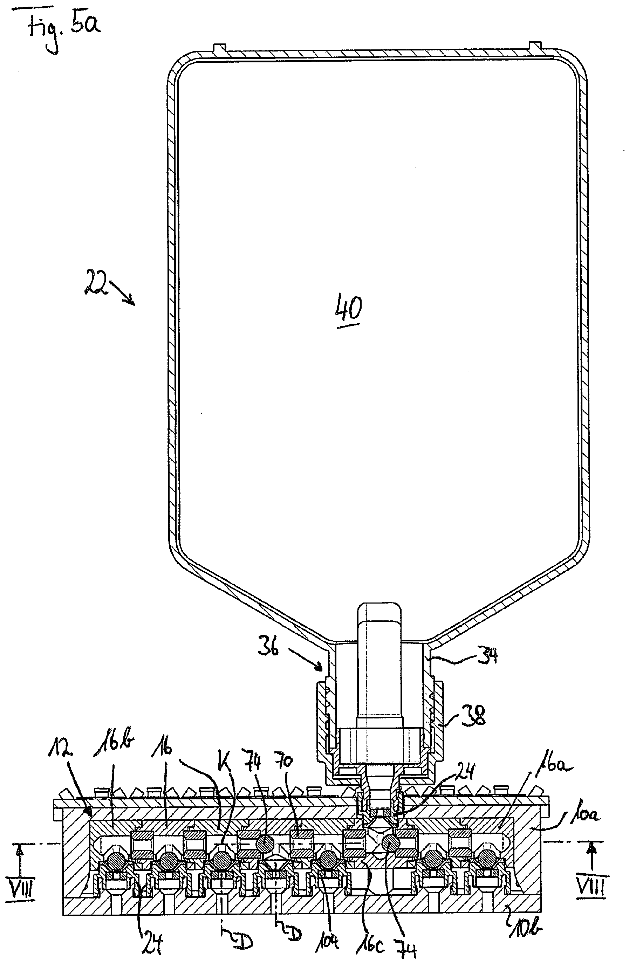

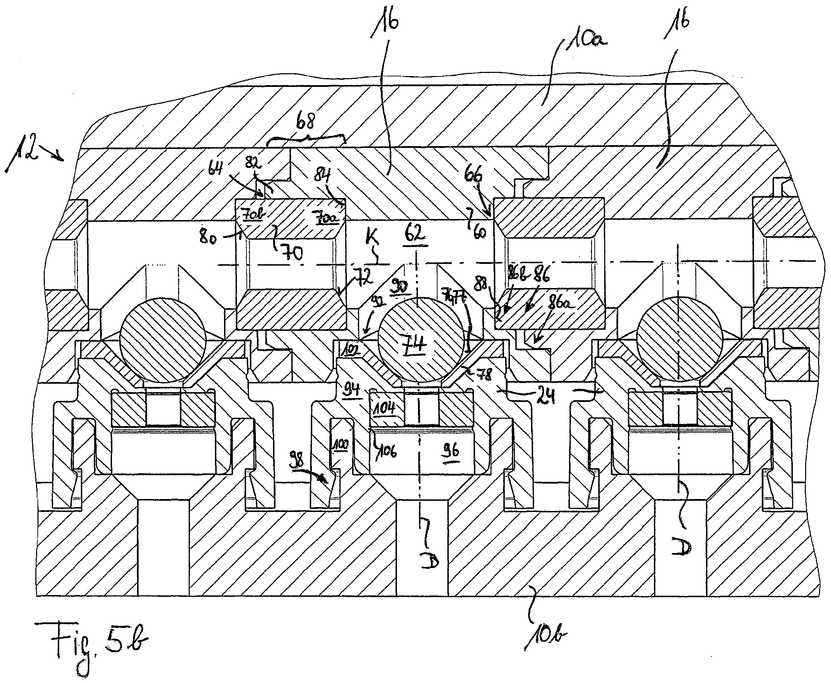

Die Fluidleitungselemente 16 können mit den Anschlussstutzen 18 mittels Fluidleitungsanschlusselementen 24 strömungsmechanisch verbunden sein. Die Fluidleitungsanordnung 12 mit den Fluidleitungselementen 16 und den Fluidleitungsanschlusselementen 24 wird weiter unten im Zusammenhang mit den Figuren 5, 7 und 8 näher erläutert werden.

Die Laborbehälteraufnahme 20 kann ein Bewegungsteil 26 umfassen, an welchem der Laborbehälter 22 vorübergehend aufgenommen werden kann. Das Bewegungsteil 26, hier in bevorzugter Form eines Schlittens, kann über eine Linearführung 28 mit der übrigen ortsfesten Tragstruktur 10 derart verbunden sein, dass das Bewegungsteil 26 und mit ihm der daran aufgenommene Laborbehälter 22 längs einer Annäherungsbahn A an die Fluidleitungsanordnung 12 annäherbar und von dieser wieder entfernbar ist.

Als Positionierungshilfe sowie zur Sicherstellung einer Kraftübertragung zwischen dem Bewegungsteil 26 und einem daran aufgenommenen Laborbehälter 22 weist das Bewegungsteil 26 eine vorzugsweise orthogonal zur Annäherungsbahn A orientierte Stirnwand 30 auf, in welcher eine Ausnehmung 32 ausgebildet sein kann, in die ein Abschnitt des Laborbehälters dann eingreifen kann, wenn dieser an dem Bewegungsteil 26 vorübergehend aufgenommen ist. Bevorzugt dient die Ausnehmung 32 der vorübergehenden Aufnahme eines Halsabschnittes 34 eines Behälterhalses 36, welcher in einem Bereich zwischen einem auf den Behälterhals 36 aufgeschraubten Behälterdeckel 38 und einem Behälterkörper 40 frei liegt.

Dann, wenn der Laborbehälter 22 am Bewegungsteil 26 aufgenommen ist, ist die Stirnwand 30 im dargelegten Beispiel formschlüssig zwischen Behälterkörper 40 und Behälterdeckel 38 mit dem freiliegenden Behälterhalsabschnitt 34 in Eingriff, so dass der Laborbehälter 22 orthogonal zur Annäherungsbahn (mit Ausnahme einer Abheberichtung aus der Ausnehmung 32 heraus) sowie in beiden Richtungen längs der Annäherungsbahn A mit der Stirnwand 30 des Bewegungsteils 26 in Eingriff ist.

Der Behälterdeckel 38 weist an dem vom Behälterkörper 40 weg weisenden Längsende eine Ventilanordnung 42 auf, welche mit der weiteren Durchströmungsöffnung 44 des Fluidleitungselements 16c durch Annäherung des Laborbehälters 22 an die Fluidleitungsanordnung 12 strömungsmechanisch ankoppelbar ist.

Eine Verbindung ist im Sinne der vorliegenden Anmeldung dann "strömungsmechanisch" hergestellt, wenn sie grundsätzlich die Durchleitung von Fluid gestattet. Dies soll jedoch nicht ausschließen, dass es Betriebszustände dieser Verbindung gibt, in welchen ein Ventilkörper eine Fluiddurchleitung durch die strömungsmechanische Verbindung verhindert.

Die Linearführung 28 ist bevorzugt mit einem Linearantrieb 46 gekoppelt, welcher für den Antrieb des Bewegungsteils 26 relativ zur übrigen Tragstruktur 10 längs der Annäherungsbahn A sorgt. Der vorzugsweise in die Linearführung integrierte Linearantrieb 46 ist mit einem in Figur 1 nicht dargestellten Steuergerät verbunden, welches eine Ansteuerung des Linearantriebs 46 zur Bewegung des Bewegungsteils 20 ermöglicht.

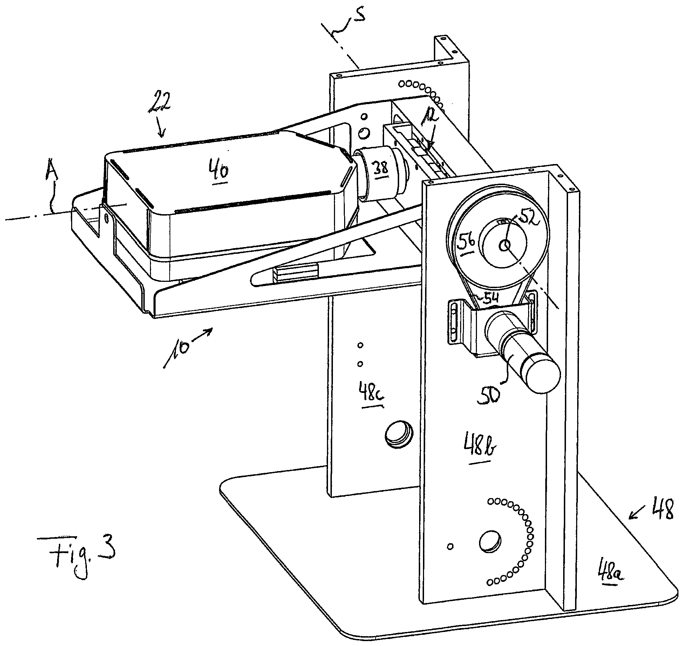

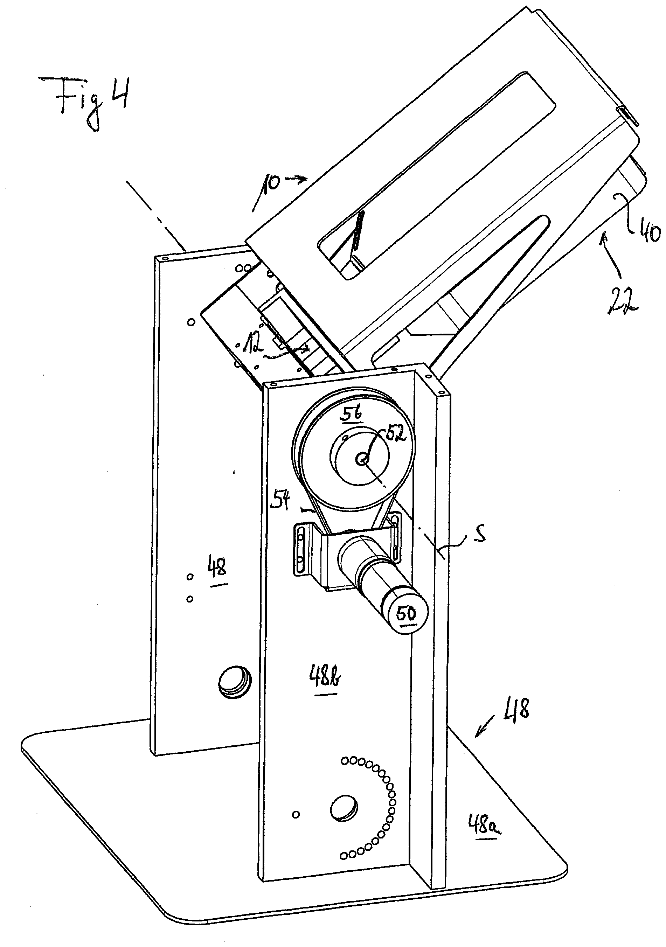

In den Figuren 3 und 4 ist die Handhabungseinrichtung der Figuren 1 und 2 mit weiteren Bauteilen dargestellt, wobei jedoch andere in den Figuren 1 und 2 gezeigte Bauteile der Übersichtlichkeit halber weggelassen sind. So zeigen die Figuren 3 und 4 beispielsweise kein Bewegungsteil 26.

In den Figuren 3 und 4 ist ein Tragrahmen 48 dargestellt, an welchem die Tragstruktur 10 der Figuren 1 und 2 um eine Schwenkachse S schwenkbar angebracht ist. Der Tragrahmen kann eine Standplatte 48a aufweisen, von welcher zwei mit Abstand voneinander angeordnete Träger 48b und 48c abstehen können. Die Träger 48b und 48c können zwischen sich die aus den Figuren 1 und 2 bekannte Tragstruktur um die Schwenkachse S schwenkbar halten.

Als Schwenkantrieb der Tragstruktur 10 kann ein elektromotorischer Antrieb 50 vorgesehen sein, welcher seine Antriebskraft in geeigneter Weise auf eine Schwenkwelle 52 überträgt, die mit der Tragstruktur 10 zur gemeinsamen Schwenkbewegung gekoppelt ist. Im in den Figuren 3 und 4 dargestellten Beispiel erfolgt die Bewegungsübertragung durch einen Riemen 54, welcher um eine drehfest mit der Schwenkwelle 52 verbundene Riemenscheibe 56 verläuft. Lediglich der Vollständigkeit halber sei darauf hingewiesen, dass die Bewegungsübertragung auch durch andere geeignete Übertragungsmittel erfolgen kann, wie etwa ein Zahnradgetriebe. Ebenfalls ist denkbar, dass die Antriebswelle des Antriebs 50 unmittelbar mit der Schwenkwelle 52 gekoppelt ist bzw. die Schwenkwelle 52 ist.

Die Figuren 3 und 4 zeigen unterschiedliche Betriebsstellungen der Handhabungseinrichtung dahingehend, dass sich die Tragstruktur 10 in beiden Figuren in einer unterschiedlichen Relativschwenkstellung zum Tragrahmen 48 befindet. Dies dient der erleichterten Benetzung von Innenwänden des Laborbehälters 22, insbesondere von dessen Behälterkörper 40, mit einer in den Laborbehälter 22 eingefüllten Flüssigkeit. So kann nach Annäherung des Laborbehälters 22 an die Fluidleitungsanordnung 12 und damit Herstellung einer strömungsmechanischen Verbindung zwischen der Fluidleitungsanordnung 12 und dem Laborbehälter 22 mittels der im Behälterdeckel 38 vorgesehenen Ventilanordnung 42 in einer ersten Betriebsstellung der Handhabungseinrichtung, etwa der Betriebsstellung von Figur 3, über die zuvor erläuterten Anschlussstutzen und geeignete Ventilschaltung (diese wird weiter unten erläutert) eine Flüssigkeit in den Laborbehälter 22 eingeleitet werden. Schwerkraftbedingt ruht diese Flüssigkeit unmittelbar nach dem Einleiten bei unveränderter Betriebsstellung - bei Einleitung in der in Figur 3 gezeigten Betriebsstellung - auf der Innenwandung der der Grundplatte 48a des Tragrahmens 48 näher gelegenen Stirnseite des Behälterkörpers 40.

Durch Schwenken des Laborbehälters 22 um die Schwenkachse S kann die in den Laborbehälter 22 eingeleitete Flüssigkeit auch mit anderen Innenflächen des Laborbehälters 22 in Kontakt gebracht werden, was insbesondere für eine Zellkultivierung in dem ebenso beispielhaft wie bevorzugt als der Laborbehälter 22 dargestellten Zellkulturbehälter vorteilhaft ist.

Der Laborbehälter 22 ist bevorzugt ein Zellkulturbehälter zur Kultivierung adhärenter Zellen.

Nachfolgend wird vor allem anhand der Figuren 5a und 5b der Aufbau der Fluidleitungsanordnung erläutert werden:
Die Figuren 5a und 5b zeigt eine Reihe von Fluidleitungselementen 16, welche miteinander sowie jeweils mit einem Fluidleitungsanschlusselement 24 strömungsmechanisch gekoppelt sind. Die Fluidleitungselemente 16 und Fluidleitungsanschlusselemente 24 sind durch eine Tragstruktur fixiert, von welcher ein fluidelementseitiger Tragstrukturteil 10a und ein fluidleitungsanschlusselementseitiger Tragstrukturteil 10b dargestellt sind. Die in den Figuren 5a und 5b gezeigten gleichartigen Fluidleitungselemente 16 sind wie folgt aufgebaut:
In einem Elementkörper 60 ist ein, vorzugsweise zylindrischer, den Elementkörper 60 durchsetzender Strömungskanal 62 ausgebildet, welcher längs einer Kanalbahn K zwischen einer ersten Durchströmungsöffnung 64 und einer zweiten Durchströmungsöffnung 66 verläuft.

In einem näher an der ersten als an der zweiten Durchströmungsöffnung gelegenen ersten Bereich 68 des Fluidleitungselements 16 ist ein Durchströmungskörper 70 vorgesehen, welcher einen Teil des Strömungskanals 62 bildet. Der Durchströmungskörper 70 ist aus einem Material gebildet, welches einen niedrigerem Elastizitätsmodul als das Material des Elementkörpers 60 aufweist, damit sich der Durchströmungskörper 70 bei Ausübung einer äußeren Kraft auf diesen in größerem Umfang verformt als der steifere, gewünscht möglichst starre Elementkörper 60. An seinem ins Innere des Elementkörpers 60 weisenden Längsende 70a weist der Durchströmungskörper 70 eine Ventilsitzformation 72 auf, beispielhaft in Form einer konischen Einsenkung. An diese kann sich ein Ventilkörper 74 anschmiegen, welcher in der in Figur 5 gezeigten Betriebssituation auf einer Ventilsitzausbildung 76 eines Sitzbauteils 78 ruht.

Damit der Durchströmungskörper 70 auch an dem in Figur 5b dem erläuterten Fluidleitungselement 16 längs der Kanalbahn K links benachbarten Fluidleitungselement als Ventilsitz dienen kann, ist auch an dem zur Außenumgebung des Fluidleitungselements 16 weisenden Längsende 70b des Durchströmungskörpers 70 eine weitere Ventilsitzformation 80 ausgebildet. Vorzugsweise ist der Durchströmungskörper 70 bezüglich einer zur Kanalbahn K orthogonalen Symmetrieebene spiegelsymmetrisch und bezüglich der Kanalbahn K als Rotationssymmetrieachse rotationssymmetrisch ausgestaltet.

Der Durchströmungskörper 70 ist vorzugsweise aus einem Elastomer-Werkstoff, etwa aus einem Kautschuk-Werkstoff oder/und einem Silikon-Werkstoff gebildet. Der Elementkörper 60 ist dagegen vorzugsweise aus einem thermoplastischen Kunststoff gebildet, wie beispielsweise aus Polypropylen, Polyehtylen oder aus Polymethylmethacrylat. Andere Kunststoffe kommen ebenfalls in Frage.

Zur erleichterten Verbindung des Fluidleitungselements 16 mit einem benachbarten Fluidleitungselement weist das in Figur 5 beispielhaft gezeigte Fluidleitungselement 16 im Bereich seiner ersten Durchströmungsöffnung 64 einen Ringbund 82 auf, welcher vom übrigen Elementkörper 60 längs der Kanalbahn K vorsteht und vorteilhafterweise den Durchströmungskörper 70 umgibt. Dadurch kann zum einen eine Positionierhilfe zur Relativpositionierung zweier axial benachbarter Fluidleitungselemente 16 geschaffen werden und kann zum anderen der Halt des Durchströmungskörpers 70 am Elementkörper 60 durch Vergrößerung der Berührfläche zwischen dem Material des Elementkörpers 60 und dem Durchströmungskörper 70 verbessert werden.

Der Durchströmungskörper 70 liegt mit seinem Längsende 70a an einem vorzugsweise vollständig um die Kanalbahn K umlaufenden Radialabsatz 84 des Elementkörpers 60 an. Auf diese Weise ist der Durchströmungskörper 70 axial bezüglich der Kanalbahn relativ zum Elementkörper 60 eindeutig positioniert.

Im Bereich der zweiten Durchströmungsöffnung 66 kann der Elementkörper 60 eine längs der Kanalbahn K in den Elementkörper 60 hinein reichende Vertiefung 86 aufweisen.

Im dargestellten Beispiel weist die Vertiefung 86 einen näher an der Außenseite des Elementkörpers 60 gelegenen ersten Ausnehmungsbereich 86a auf, welcher zur Aufnahme eines Ringbunds 82 eines benachbarten Fluidleitungselements 16 geeignet ist, um dieses relativ zum Fluidleitungselement 16 bezüglich der Kanalbahn K zu positionieren. Bevorzugt weist die Innenumfangsfläche des ersten Ausnehmungsbereichs 86a eine bezüglich einer Außenumfangsfläche eines Ringbunds eines benachbarten gleichartigen Fluidleitungselements 16 eine komplementäre Gestalt auf, so dass zwei Fluidleitungselemente 16 durch Zusammenstecken, genauer: durch Einführen des Ringbundes 82 in den ersten Ausnehmungsbereich 86a des Fluidleitungselements 16, mit kollinearen Kanalbahnen K derart verbindbar sind, dass ihre beiden Strömungskanäle 62 einen gemeinsamen langen Strömungskanal bilden.

Die Vertiefung 86 kann weiter einen zweiten Ausnehmungsbereich 86b aufweisen, welcher in Richtung in den Elementkörper 60 hinein weiter von der Außenseite des Elementkörpers 60 entfernt gelegen ist. Dieser zweite Ausnehmungsbereich 86b dient der Aufnahme eines axial auskragenden Längsendes 70b eines benachbarten Fluidleitungselements 16, um zwei Fluidleitungselemente 16 fluiddicht miteinander zu verbinden. Hierzu kann der zweite Ausnehmungsbereich 86b einen Radialvorsprung 88 aufweisen, an dem eine Stirnseite des vom benachbarten Fluidleitungselement 16 auskragenden Durchströmungskörpers 70 im fertig montierten Zustand anliegt.

Somit liegt im fertig montierten Zustand der Fluidleitungsanordnung 22 ein Durchströmungskörper 70 mit seinen beiden entgegengesetzten Stirnseiten an zwei entgegengesetzten radialen Vorsprüngen 84 und 88 an, welche an zwei verschiedenen Fluidleitungselementen 16 ausgebildet sind.

Bevorzugt sind die Vertiefung 86, insbesondere deren zweiter Ausnehmungsbereich 86b mit dem Radialvorsprung 88, sowie der Radialvorsprung 84 derart angeordnet, dass sie im fertig montierten Zustand einen geringeren Abstand voneinander aufweisen als die Abmessung des Durchströmungskörpers 70 längs der Kanalbahn im unbelasteten Zustand. Beim Montieren der Fluidleitungsanordnung 12 kommt es daher zu einer geringfügigen axialen Kompression des Durchströmungskörpers 70 zwischen den beiden ihm zugeordneten radialen Vorsprüngen 84 und 88, was die Dichtigkeit der unter Beteiligung des Durchströmungskörpers 70 gebildeten Verbindung zwischen zwei axial benachbarten Fluidleitungselementen 16 erhöht.

Nachzutragen ist, dass die Innenumfangsfläche des zweiten Ausnehmungsbereichs 86b komplementär zur Außenumfangsfläche des auskragenden Längsendbereichs eines Durchströmungskörpers 70 ausgebildet ist, so dass auch der Außenumfangsbereich des auskragenden Abschnitts eines Durchströmungskörpers 70 nach Einführen in den zweiten Ausnehmungsbereich 86b eines benachbarten Fluidleitungselements 16 in Anlage an dessen Außenumfangsfläche im zweiten Ausnehmungsbereich 86b gelangt, was wiederum die Dichtungswirkung des Durchströmungskörpers 70 verbessert.

Dadurch, dass der einem Fluidleitungselement 16 zugeordnete Strömungskörper 70 im Bereich der ersten Durchströmungsöffnung über eine wesentlich größere Berührfläche in Berührkontakt mit dem zugeordneten Elementkörper 60 steht als mit der Innenumfangsfläche eines benachbarten Fluidleitungselements, verbleibt der Durchströmungskörper 70 bei einer Demontage der Fluidleitungsanordnung 12 in dem ihm zugeordneten Fluidleitungselement 16.

Die in den Figuren 5a und 5b gezeigten Fluidleitungselemente 16 weisen neben dem Strömungskanal 62 einen davon abzweigenden Zusatz-Strömungskanal 90 auf. Dieser mündet in einer weiteren Durchströmungsöffnung 92. Vorzugsweise ist der Zusatz-Strömungskanal 90 geradlinig und zweigt im rechten Winkel vom Strömungskanal 62 ab.

Mit der weiteren Durchströmungsöffnung 72 kann ein Fluidleitungsanschlusselement 24 verbunden sein, welches bevorzugt an dem Tragstrukturteil 10b gehaltert ist.

Das Fluidleitungsanschlusselement 24 weist eine Elementbasisgestalt 94 auf, welche längs einer Durchlassbahn D von einem Durchströmkanal 96 durchsetzt ist.

Mit einer Anbringungsausbildung 98, vorzugsweise ausgestaltet als umlaufender radial nach innen zur Durchlassbahn D vorstehender und radial federnder Rastvorsprung ist das Fluidleitungsanschlusselement an einer Gegenausbildung 100 der Tragstruktur 10, genauer: des Tragstrukturteils 10b, durch Verrastung fixiert.

Die Anbringungsausbildung 98 ist an einem Längsende des Fluidleitungsanschlusselements 24 ausgebildet.

An dem diesem entgegengesetzten Längsende ist gemäß einer bevorzugten Ausführungsform der vorliegenden Erfindung das oben bereits erwähnte elastomere Sitzbauteil 78 mit der Ventilsitzausbildung 76 angeordnet. Das Sitzbauteil 78 bildet somit bevorzugt ein Längsende des Fluidleitungsanschlusselements 24 in dem dargestellten bevorzugten Ausführungsbeispiel.

Das Sitzbauteil 78 weist neben der Ventilsitzausbildung 76 einen Dichtungsabschnitt 102 auf, welcher bevorzugt die Ventilsitzausbildung 76 bzw. den die Ventilsitzausbildung 76 aufweisenden Sitzabschnitt 77 des Sitzbauteils 78 radial außen umgibt. Der Dichtungsabschnitt 102 ist im fertig montierten Zustand vorzugsweise zwischen einem die weitere Durchströmungsöffnung 92 umgebenden Abschnitt des Elementkörpers 60 und einem den Durchströmkanal 96 umgebenden Abschnitt der Elementbasisgestalt 94 geklemmt, um die Verbindung zwischen dem Zusatz-Strömungskanal 90 des Fluidleitungselements 16 und dem Durchströmkanal 96 des Fluidleitungsanschlusselements 24 abzudichten.

Das Fluidleitungsanschlusselement 24 umfasst weiter einen Permanentmagneten 104, welcher vorzugsweise einen Teil des Durchströmungskanals umgibt, besonders bevorzugt einen Teil desselben bildet.

Im vorliegend dargestellten Beispiel ist der Permanentmagnet 104 von dem der Anbringungsausbildung 98 näher gelegenen Längsende des Durchströmkanals 96 her in diesen längs der Durchlassbahn D eingeschoben und an einer radialen Rastnase 106 im Durchströmkanal verrastet. Der Permanentmagnet 104 bildet somit eine Querschnittsverengung des Durchströmkanals 96.

Der Permanentmagnet 104 gestattet die Verwendung von ferromagnetischen Ventilkörpern 74, bevorzugt in der Form einer Ventilkugel. Ohne weiteren Einfluss von außen sitzt daher der Ventilkörper 74 bzw. die Ventilkugel 74 aufgrund des vom Permanentmagneten 104 ausgehenden Magnetfelds auf der Ventilsitzausbildung 76 des Sitzbauteils 78. Der Durchströmkanal 96 ist dann vom Ventilkörper 74 für einen Fluiddurchlass gesperrt.

Figur 6 zeigt eine perspektivische Ansicht einer Ventilleitungsanordnung mit daran angekoppeltem Laborbehälter 22. Die Ventilleitungsanordnung weist die zuvor beschriebene Fluidleitungsanordnung 12 und eine Schaltanordnung 14 auf. Die Schaltanordnung 14 umfasst eine Mehrzahl von Schalteinheiten 110, die im Wesentlichen gleich aufgebaut sind, so dass nachfolgend nur eine einzige Schalteinheit 110 stellvertretend für die übrigen Schalteinheiten beschrieben werden wird. Die Schalteinheit 110 weist einen längs einer Schaltbewegungsbahn B verlagerbaren Permanentmagneten 112 auf. Genauer ist der Permanentmagnet 112 längs der Schaltbewegungsbahn an ein Fluidleitungselement 16 annäherbar und von diesem wieder entfernbar. Die Schaltbewegungsbahn B der Schalteinheiten 110 ist bevorzugt orthogonal zur Kanalbahn K und ihre gedachte Verlängerung schneidet diese.

Der Permanentmagnet 112 einer Schalteinheit ist vorzugsweise in einem Führungsgehäuse 114 zur Bewegung längs der Schaltbewegungsbahn B geführt. Außerdem schirmt das Führungsgehäuse 114 den Permanentmagneten 112 vor Umwelteinflüssen ab.

In dem in den Figuren 6 bis 8 dargestellten Ausführungsbeispiel schließt sich an das Führungsgehäuse 114, welches einenends an der die Fluidleitungselemente 16 aufnehmenden Tragstruktur endet, andernends eine pneumatische Antriebseinrichtung 116 an. Die Antriebseinrichtung 116 umfasst einen ortsfesten, vorzugsweise mit dem Führungsgehäuse 114 verbundenen Zylinder 118 und eine im Zylinder längs der Schaltbewegungsbahn B relativ zum Zylinder 118 bewegliche Kolbenstange 120. Die Kolbenstange 120 ist vorzugsweise doppeltwirkend ausgebildet, so dass durch gezielte Druckeinleitung auf einer von zwei Seiten des Kolbens 122 eine gezielte Annäherung des Permanentmagneten 112 an das ihm zugeordnete Fluidleitungselement 16 und eine Entfernung von diesem bewirkbar ist.

Durch die Annäherung des Permanentmagneten 112 an das ihm zugeordnete Fluidleitungselement 16 und durch die Entfernung von diesem kann in einem Schaltbereich 124, welcher um die Schnittstelle zwischen Schaltbewegungsbahn und Kanalbahn herum besteht, die Magnetfeldstärke des vom Permanentmagneten 112 ausgehenden Magnetfelds verändert werden.

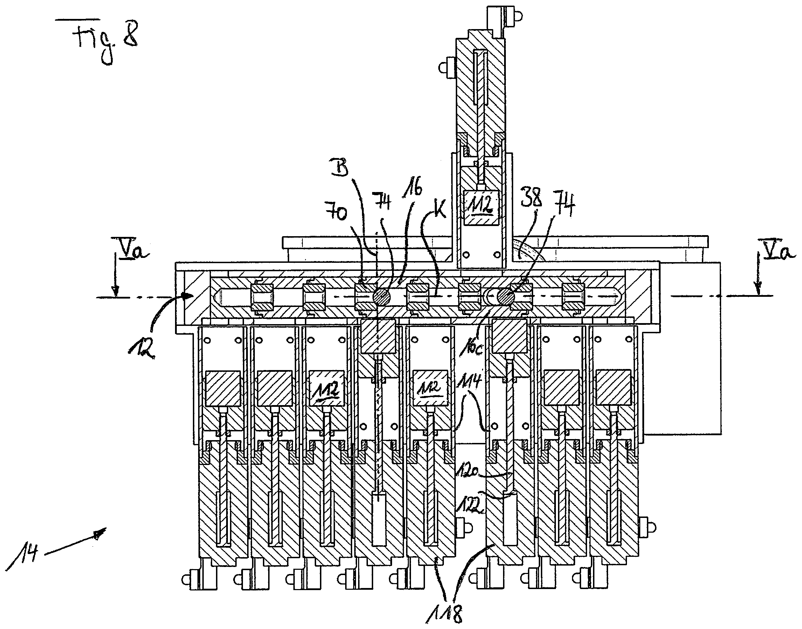

Wie in Figur 8 erkennbar ist, durchsetzt die zum zugeordneten Fluidleitungselement 16 hin verlängert gedachte Schaltbewegungsbahn B den Strömungskanal 62 des zugeordneten Fluidleitungselements 62 bevorzugt zwischen dem im Inneren des Elementkörpers 60 liegenden Längsende 70a des Durchströmungskörpers 70 und dem Ort der größten zur Kanalbahn K orthogonalen Ausdehnung des an der im Inneren des Elementkörpers 60 gelegenen Ventilformation 72 anliegenden Ventilkörpers 74. Dadurch ist gewährleistet, dass ein Absenken des dieser Ventilsitzformation 72 zugeordneten Permanentmagneten 122 und die damit verbundene Erhöhung der Magnetfeldstärke im zugeordneten Schaltbereich 124 eine Verlagerung des Ventilkörpers 74 von der Ventilsitzausbildung 76 weg zur Anlage an die Ventilsitzformation 72 bewirkt. Hier ist es alternativ auch möglich, dass die in gleicher Weise verlängert gedachte Schaltbewegungsbahn B den Durchströmungskörper 70 durchsetzt, etwa nahe bei der Ventilsitzformation 72. Hier hat der Konstrukteur also einen gewissen Spielraum bei der Anordnung der Schalteinheit 110. Dieser Spielraum hängt auch von der Gestalt und Stärke der verwendeten Permanentmagneten 112 ab. Es wird für den Durchschnittsfachmann jedoch keinerlei Probleme darstellen, durch zumutbaren Versuchsaufwand für einen gegebenen Permanentmagneten in der Annäherungsposition den geeigneten Anbringungsort im Einzelfall zu ermitteln.

Das eine Anschlussmöglichkeit für Laborbehälter bereitstellende Fluidleitungselement 16c weist im dargestellten Ausführungsbeispiel als einziges Fluidelement sowohl an der ersten Durchströmungsöffnung 64 als auch an der zweiten Durchströmungsöffnung 66 je einen Durchströmungskörper auf. Somit weist dieses Fluidleitungselement 16c zwei im Inneren seines Elementkörpers 60 gelegene und zum Strömungsraum des Fluidleitungselements 16c hinweisende Ventilsitzformationen auf. Jeder dieser Ventilsitzformationen ist je eine Schalteinheit 110 zugeordnet, die aus Platzgründen auf unterschiedlichen, vorzugsweise entgegengesetzten Seiten der Fluidleitungsanordnung 12 vorgesehen sind. Bei entsprechend schlankerer Ausbildung der Permanentmagneten 112 könnten jedoch auch diese beiden Schalteinheiten 110 auf einer gemeinsamen Seite der Fluidleitungsanordnung 12 gelegen sein.

Die endseitigen Fluidleitungselemente 16a und 16b weisen im dargestellten Beispiel lediglich einen um 90° abgewinkelten Strömungsraum, aber keinen Zusatz-Strömungskanal auf.

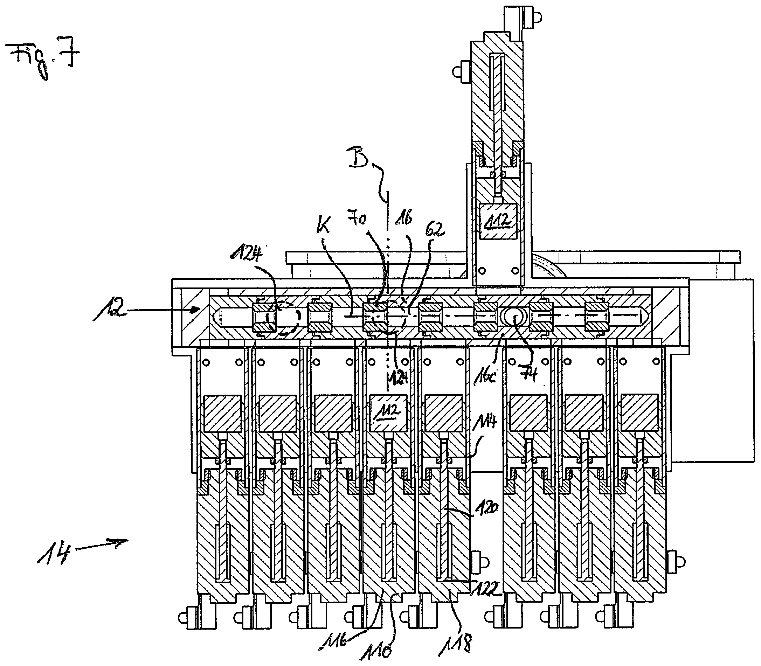

In Figur 7 ist die Schaltanordnung 14 mit allen Schalteinheiten 110 in der Inaktivstellung gezeigt, d. h. keiner der vorhandenen Permanentmagneten 112 ist an die Fluidleitungsanordnung 12 angenähert. Folglich befinden sich alle vorhandenen Ventilkörper 74 an den Ventilsitzausbildungen 76 der mit den jeweiligen Fluidleitungselementen 16 verbundenen Fluidleitungsanschlusselemente 24. Diese Anlage an den Ventilsitzausbildungen 76 wird bei dem in Figur 7 gezeigten inaktiven Zustand der Schaltanordnung 14 durch die jeweiligen Permanentmagnete 104 in den Durchströmkanälen 96 der Fluidleitungsabschlusselemente 24 bewirkt.

Dann jedoch, wenn - wie in Figur 8 gezeigt - einzelne Permanentmagnete 112 zu den ihnen zugeordneten Fluidleitungselementen 16 bzw. den ihnen zugeordneten Ventilsitzformationen 72 hin abgesenkt sind, ist in dem ebenfalls zugeordneten jeweiligen Schaltbereich 124 das aufgrund des Permanentmagneten 112 wirkende Magnetfeld so stark, dass der ferromagnetische Ventilkörper 74 vom Permanentmagneten 104 des Fluidleitungsanschlusselements 24 und damit von der Ventilsitzausbildung 76 weg zu der zugeordneten Ventilsitzformation 72 hin verlagert wird.

In der in Fig. 7 gezeigten Betriebsstellung der Schaltanordnung 14 sind alle Anschlussleitungen über die Anschlussstutzen 18 sowie die in den Laborbehälter 22 führende Leitung durch je einen Ventilkörper verschlossen.

Dagegen ist in der Betriebsstellung von Figur 8 die in den Behälter führende Leitung geöffnet. Außerdem ist die Anschlussleitung des bei Betrachtung der Figur 8 zweiten Fluidleitungselements 16 zur rechten des mit dem Laborbehälter 22 gekoppelten Fluidleitungselements 16c geöffnet. Durch geeigneten Druckaufbau in der zugehörigen Anschlussleitung, welche für eine Durchströmung freigegeben ist, kann somit entweder ein Fluid in den Laborbehälter 22 eingeleitet oder aus diesem entnommen werden.

Zur besseren Orientierung sei darauf hingewiesen, dass die aus den Figuren 1 und 2 bekannten Anschlussstutzen vor der Zeichenebene der Figuren 7 und 8 gelegen sind. Auch die mit den jeweiligen Fluidleitungselementen 16 in den Figuren 7 und 8 verbundenen Fluidleitungsanschlusselemente 24 liegen vor der Zeichenebene der Figuren 7 und 8 und damit vor der Schnittebene der dort gezeigten Schnittdarstellung. Daher sind die Fluidleitungsanschlusselemente 24 in den Figuren 7 und 8 nicht zu erkennen.

Durch geeignete Schaltung der durch die Fluidleitungsanschlusselemente 24, Fluidleitungselemente 16 und Ventilkörper 74 gebildeten ventilbehafteten Fluidleitungsstrecken können nicht nur unterschiedliche Fluide in den angeschlossenen Laborbehälter 22 eingeleitet und aus diesem entnommen werden, sondern es kann auch der kombinierte Strömungskanal der Fluidleitungsanordnung 12 gereinigt werden, etwa durch Hindurchleiten eines Reinigungsfluids durch die Fluidleitungsstrecke.

Mit der vorliegenden Erfindung ist es also möglich, mehrere Laborbehälter aus einer einzigen Vorratsquelle mit einem jeweiligen Fluid zu versorgen bzw. verbrauchte Fluide aus einer Mehrzahl von Laborbehältern zu entsorgen, ohne dass dadurch eine Kreuzkontamination der Laborbehälterinhalte zu befürchten wäre. Versuche haben nämlich gezeigt, dass die Reinigungsspülung durch die erfindungsgemäß modular aufgebaute Fluidleitungsanordnung 12 höchsten Reinheitsanforderungen genügt.

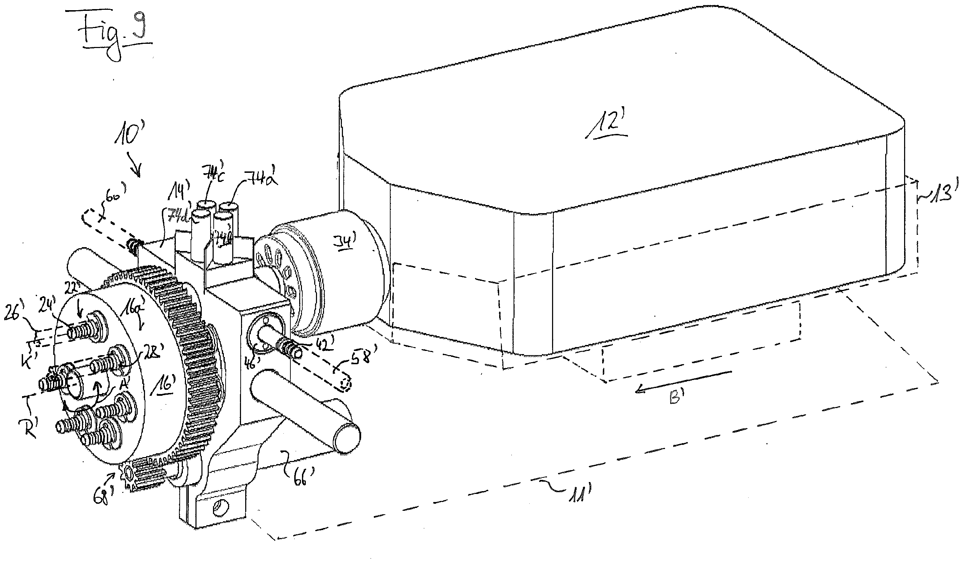

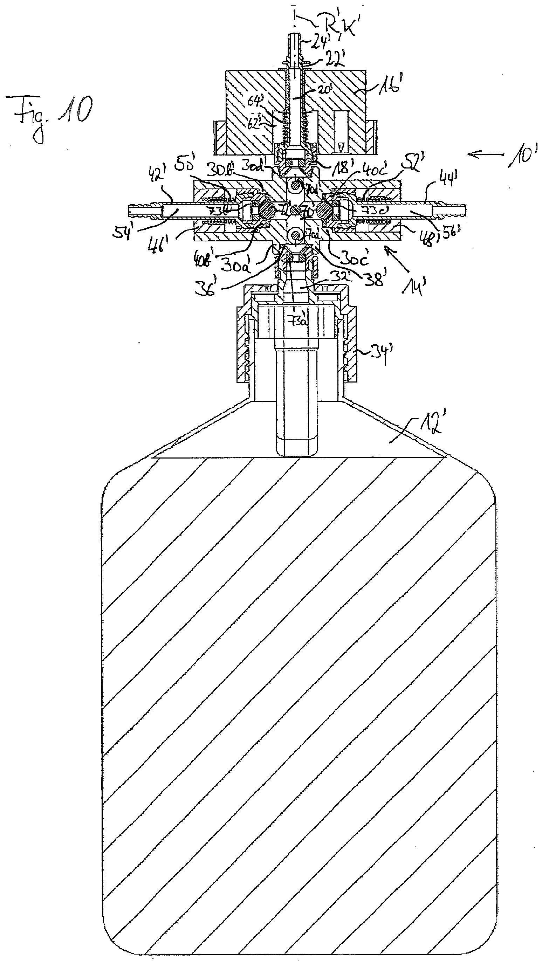

In den Figuren 9 und 10 ist eine zweite Ausführungsform einer erfindungsgemäßen Handhabungseinrichtung gezeigt. Die zur Beschreibung der zweiten Ausführungsform verwendeten Bezugszeichen sind zur Unterscheidung von den Bezugszeichen der ersten Ausführungsform mit einem Apostroph versehen.

In den Figuren 9 und 10 ist eine an einer Tragstruktur 11' aufgenommene Ventilleitungsanordnung allgemein mit 10' bezeichnet. An diese Ventilleitungsanordnung 10' ist in den Figuren 9 und 10 ein beispielsweise als Zellkulturbehälter ausgebildeter Laborbehälter 12' angekoppelt.

Die Ventilleitungsanordnung 10' umfasst ein Leitungsbauteil 14', an welchem ein Fluidkanalträger 16' um eine Rotationsachse R' drehbar vorgesehen ist.

An dem Fluidkanalträger 16' ist eine Mehrzahl - im vorliegenden Beispiel genau sechs - von Fluidkanalträger-Anschlussformationen 18' (siehe Figur 10) aufgenommen, welche jeweils von einem Fluidkanalabschnitt 20' durchsetzt sind.

Die Fluidkanalträger-Anschlussformation 18', wie die übrigen Fluidkanalträger-Anschlussformationen 19' auch, ist an einem Fluidkanalträger-Anschlussstutzen 22' ausgebildet, welcher an seinem von der Fluidkanalträger-Anschlussformation 18' fernliegenden Längsende 24' zum Anschluss eines Fluidleitungsmittels 26', beispielsweise in Form eines flexiblen Schlauchs, ausgebildet ist.

In Figur 9 ist zur Unterscheidung der Fluidkanalträger-Anschlussstutzen 22' voneinander ein weiterer Anschlussstutzen mit 28' bezeichnet. Die Fluidkanalträger-Anschlussstutzen sind bevorzugt im Wesentlichen identisch aufgebaut.

Wie man in Figur 10 erkennt, weist das Leitungsbauteil 14' eine Mehrzahl von - im vorliegenden Beispiel vier - Ankopplungsformationen 30a' bis 30d' auf. Die Ankopplungsformation 30a' ist dabei zur vorübergehenden Ankopplung eines Fluidkanals 32' ausgebildet, der in einem Deckel 34' des Zellkulturbehälters 12' in an sich bekannter Weise vorgesehen ist. Der Deckel 34' weist zur Ankopplung an die Ankopplungsformation 30a' eine Zellkulturbehälter-Anschlussformation 36' auf, welche im Wesentlichen vorteilhaft in Übereinstimmung mit den übrigen Anschlussformationen, beispielsweise der Fluidkanalträger-Anschlussformation 18', ausgebildet ist.

Die Ankopplungsformationen 30a' bis 30d' weisen jeweils bevorzugt einen umlaufenden Bund auf, in welchen die zugeordnete Anschlussformation zur Ankopplung an die jeweilige Ankopplungsformation 30a' bis 30d' eingetaucht ist. Der Übersichtlichkeit halber ist lediglich der Bund 38' der Ankopplungsformation 30a' mit einem Bezugszeichen versehen.

Die Ankopplungsformationen 30b' und 30c' sind im vorliegenden Beispiel fest und dauerhaft mit Anschlussformationen 40b' und 40c' zur Bildung einer gemeinsamen Fluidleitungsstrecke gekoppelt. Hierzu kann das Leitungsbauteil 14' einen von Fluidkanalstutzen 42' und 44' durchsetzten Stützkörper 46' bzw. 48' aufweisen, an welchem sich eine Spannfeder 50' bzw. 52' abstützt, die die Anschlussformationen 40b' bzw. 40c' zu den jeweiligen Ankopplungsformationen 30b' bzw. 30c' hin vorspannt. Die Stützkörper 46' und 48' sind hierzu als Federwiderlager bevorzugt fest mit dem Leitungsbauteil 14' verbunden.

Die Anschlussformationen 40b' und 40c' sind von einem Fluidkanalabschnitt 54' bzw. 56' durchsetzt, welcher von den Anschlussstutzen 42' und 44' definiert sind, die an ihren von der Anschlussformation 40b' bzw. 40c' jeweils fernliegenden Längsenden wiederum zur Anbringung je eines Fluidleitungsmittels - hier eines Schlauchs 58' bzw. 60' - ausgebildet sind.

Im Gegensatz zu den fest mit den Ankopplungsformationen 30b' und 30c' gekoppelten Anschlussformationen 40b' und 40c' kann die Ankopplungsformation 30d' mit unterschiedlichen Anschlussformationen des Fluidkanalträgers 16' zur Herstellung einer gemeinsamen Fluidleitungsstrecke gekoppelt werden. Die Ankopplungsformation 30d' wird daher nachfolgend als Wechsel-Ankopplungsformation 30d' bezeichnet.

Da in den Figuren 9 und 10 die nur in Figur 10 erkennbare Fluidkanalträger-Anschlussformation 18' des Fluidkanalträgers 16' der Wechsel-Ankopplungsformation 30d' am stärksten angenähert ist, mithin diese als Kopplungs-Anschlussformation 18' zur Kopplung mit der Wechsel-Ankopplungsformation 30d' ausgewählt ist, ist nachfolgend die Fluidkanalträger-Anschlussformation 18' als Kopplungs-Anschlussformation 18' bezeichnet. Diese befindet sich in Figur 10 in einer Kopplungsstellung, in welcher sie mit der Wechsel-Ankopplungsformation 30d' zur Bildung einer gemeinsamen Fluidleitungsstrecke, die dann die Kopplungs-Anschlussformation 18' und die Wechsel-Ankopplungsformation 30d' durchsetzt, gekoppelt ist.

Die Kopplungs-Anschlussformation 18' von Figur 10 ist längs einer zur Rotationsachse R' vorzugsweise parallelen Kopplungsbahn K', insbesondere Kopplungsachse K', zwischen der in Figur 10 gezeigten Kopplungsstellung und einer Kopplungsbereitschaftsstellung verstellbar, in welcher die Kopplungs-Anschlussformation 18' längs der Kopplungsbahn K' mit Abstand von der Wechsel-Ankopplungsformation 30d' angeordnet ist. Die Kopplungs-Anschlussformation 18' befindet sich dann tiefer in dem koaxialen Aufnahmeraum 62' im Fluidkanalträger 16' aufgenommen, so dass der Fluidkanalträger 16' um die Rotationsachse R' rotiert werden kann, ohne dass eine Kollision zwischen der Kopplungs-Anschlussformation 18' oder einer anderen Fluidkanalträger-Anschlussformation mit der Wechsel-Ankopplungsformation 30d' zu befürchten ist. In der Kopplungsbereitschaftsstellung fluchtet die Kopplungs-Anschlussformation 18' bevorzugt immer noch mit der Wechsel-Ankopplungsformation 30d', so dass die Kopplungs-Anschlussformation 18' durch Verlagerung derselben längs der Kopplungsbahn K' auf die Wechsel-Ankopplungsformation 30d' zu in einfacher Weise translatorisch in die Kopplungsstellung überführt werden kann.

Die Kopplungs-Anschlussformation 18' kann - wie die übrigen Fluidkanalträger-Anschlussformationen auch - durch eine Vorspanneinrichtung, etwa eine Schraubenfeder 64', längs der Kopplungsbahn K' in die Kopplungsstellung vorgespannt sein. In diesem Falle weist der Fluidkanalträger 16' ein in den Figuren 9 und 10 nicht dargestelltes Kraftgerät auf, welche alle Fluidkanalträger-Anschlussformationen gegen die Wirkung der ihnen jeweils zugeordneten Druckfeder in die ihnen ebenfalls jeweils zugeordneten Aufnahmeräume zurückzieht. Bevorzugt können die Fluidkanalträger-Anschlussformationen oder die sie tragenden Anschlussstutzen in der in den Fluidkanalträger 16' zurückgezogenen Stellung verriegelt werden, beispielsweise durch eine Bajonettformation.

Alternativ können die Fluidkanalträger-Anschlussformationen auch in die jeweilige in den Aufnahmeraum 62' zurückgezogene Stellung vorgespannt sein, etwa wenn die Schraubenfeder 64' eine Zugfeder ist. In diesem Falle kann zumindest die jeweils zur Ankopplung mit der Wechsel-Ankopplungsformation 30d' ausgewählte Kopplungs-Anschlussformation 18' durch ein in den Figuren 9 und 10 nicht dargestelltes Kraftgerät gegen die Wirkung der Vorspannfeder 64' von der Kopplungsbereitschaftsstellung in die in Figur 10 gezeigte Kopplungsstellung verstellt und gegebenenfalls in der Kopplungsstellung gegen eine Zurückbewegung in die Kopplungsbereitschaftsstellung verriegelt werden.

Eine der Fluidkanalträger-Anschlussformationen (siehe Anschlussstutzen 22' und 28' in Figur 9) kann durch Rotation des Fluidkanalträgers 16' relativ zum Leitungsbauteil 14' als Kopplungs-Anschlussformation ausgewählt werden, indem die jeweilige Fluidkanalträger-Anschlussformation längs einer Kreisbahn A' als einer Auswahlbahn um die Rotationsachse R' bewegt wird, bis die ausgewählte Kopplungs-Anschlussformation mit der Wechsel-Ankopplungsformation 30d' fluchtet und sich somit in einer Kopplungsbereitschaftsstellung befindet.

Zur Erleichterung der Bewegung der Fluidkanalträger-Anschlussformationen um die Rotationsachse R' ist bevorzugt ein Bewegungsantrieb 66', besonders bevorzugt ein elektromotorischer Bewegungsantrieb 66', am Leitungsbauteil 14' vorgesehen, welcher Bewegung und Kraft übertragend mit dem Fluidkanalträger 16' gekoppelt ist, beispielsweise über ein Zahnradgetriebe 68'. Hierzu kann ein Teil der Außenumfangsfläche 16a' (Mantelfläche) des Fluidkanalträgers 16' als Zahnrad oder Zahnkranz ausgebildet sein, vorzugsweise einstückig durch Kunststoffspritzguss.

Im Leitungsbauteil 14' ist eine Strömungskanalanordnung 70' vorgesehen, die die jeweiligen Ankopplungsformationen 30a' bis 30d' durchsetzenden Fluidleitungsabschnitte 70a' bis 70d' (aus Gründen besserer Übersichtlichkeit sind nur die Fluidleitungsabschnitte 70a' und 70d' mit Bezugszeichen versehen) miteinander fluidleitend verbindet. Die nicht eigens gekennzeichneten Fluidleitungsabschnitte 70b' und 70c' durchsetzen die mit gleichen Kleinbuchstaben gekennzeichneten Ankopplungsformationen 30b' bzw. 30c'.

Die Strömungskanalanordnung 70' verbindet die Fluidleitungsabschnitte 70a', 70b', 70c' und 70d' im dargestellten Beispiel der Figur 10 parallel zueinander in einer sogenannten Sternform. In dieser Ausgestaltung können jeweils zwei der Fluidleitungsabschnitte 70a' bis 70d' miteinander fluidleitend verbunden sein, ohne dass die Fluidleitung an den übrigen Fluidleitungsabschnitten entlang- oder an den Ankopplungsformationen vorbeiströmen muss.

Die dauerhaft mit den Ankopplungsformationen 30b' und 30c' gekoppelten Anschlussformationen 40b' und 40c' tragen in Figur 10 vorzugsweise kugelförmige Ventilkörper 72' (nur der Ventilkörper 72' an der Anschlussformation 40d' ist mit einem Bezugszeichen versehen), welche bevorzugt ferromagnetisches Material umfassen oder aus ferromagnetischem Material gebildet sind. Das Leitungsbauteil 14' kann in an sich bekannter Weise verlagerbare Schaltmagnete aufweisen, welche in Zylinderführungen 74a' bis 74d' zur Annäherung an und Entfernung von den Ventilkörpern 72' geführt sein können, um die Ventilkörper 72' innerhalb der Strömungskanalanordnung 70' durch Veränderung des lokal auf sie einwirkenden Magnetfelds zu verlagern. Die in Figur 10 gezeigten zwei Ventilkörper bei vier Ankopplungsformationen 30a' bis 30d' sind ausreichend, um stets zwei Ankopplungsformationen und damit die sie durchsetzenden Fluidleitungsabschnitte in fluidleitender Kommunikation zu halten, während die übrigen zwei Ankopplungsformationen für eine Fluidleitung gesperrt sind. In Abwesenheit eines Magnetfelds von Schaltmagneten sind die Ventilkörper 72' durch Magnete 73a' bis 73d' in den Ventilsitzen in die jeweilige Schließstellung vorgespannt.

Beispielsweise kann die Fluidleitung 58' mit einem Entsorgungsbehälter gekoppelt sein und kann die Fluidleitung 60' mit einem Vorratsbehälter für Reinigungsfluid gekoppelt sein.

Die Fluidkanalstutzen 22', 28' sowie die nicht weiter mit Bezugszeichen versehenen Fluidkanalstutzen der Figur 9 können mit unterschiedlichen Vorratsbehältern für Medien gekoppelt sein, beispielsweise mit einem Vorratsbehälter für Nährmedium, mit einem Vorratsbehälter für Medium zum Ablösen adhärenter Zellen von Innenflächen des Zellkulturbehälters und dergleichen.

Wie in Figur 9 dargestellt, kann die Handhabungseinrichtung zum Ankoppeln des Laborbehälters 12' an die Ventilleitungsanordnung 10' ein gestrichelt dargestelltes Bewegungsteil 13' aufweisen. Dieses kann beispielsweise als Schlitten oder Wagen ausgebildet sein. Das Bewegungsteil 13' ist zur vorübergehenden Aufnahme des Laborbehälters 12' ausgebildet und ist längs einer Anschlussbahn B' an die Ventilleitungsanordnung 10' annäherbar und von dieser entfernbar.

Um die Innenflächen des Laborbehälters 12' mit einer in den Laborbehälter 12' eingeleiteten Flüssigkeit gezielt benetzen zu können, kann vorgesehen sein, dass das Bewegungsteil 13' und die Ventilleitungsanordnung 10' wenigstens dann, wenn sich das Bewegungsteil 13' in einer an die Ventilleitungsanordnung 10' angenäherten Stellung befindet, um eine gemeinsame Schwenkachse schwenkbar an einem nicht dargestellten Tragrahmen der Handhabungseinrichtung vorgesehen sind. Wie bei der ersten Ausführungsform kann auch bei der zweiten Ausführungsform als Schwenkantrieb ein elektromotorischer Antrieb vorgesehen sein. Die weiteren diesbezüglichen Ausführungen zur ersten Ausführungsform gelten im Übrigen auch für die zweite Ausführungsform.

## Patentansprüche

1. Handhabungseinrichtung mit einer Ventilleitungsanordnung (10'), insbesondere für eine Zellkulturanlage, zur Einleitung eines Fluids in einen Laborbehälter (22; 12'), insbesondere Zellkulturbehälter, oder/und zur Ausleitung eines Fluids aus diesem und mit einer die Ventilleitungsanordnung aufnehmenden Tragstruktur (10; 11'), die Ventilleitungsanordnung (10') umfassend ein Leitungsbauteil (12; 14')
- mit einer ersten Ankopplungsformation (44; 30a') zur vorübergehenden Ankopplung eines ersten Fluidkanals (32'), vorzugsweise eines Fluidkanals eines Laborbehälters (22; 12'),
- mit einer zweiten Ankopplungsformation (18, 24; 30d') zur vorübergehenden oder dauerhaften Ankopplung eines zweiten Fluidkanals (20'), vorzugsweise eines Fluidkanals eines Fluidvorrats, und
- mit einer dritten Ankopplungsformation (18, 24; 30b', 30c') zur vorübergehenden oder dauerhaften Ankopplung eines dritten Fluidkanals (54', 56'), vorzugsweise eines Entsorgungskanals,
welche Ankopplungsformationen (44, 18, 24; 30a'-30d') jeweils von einem Fluidleitungsabschnitt (bei 44, 96; 70a'-70d') durchsetzt sind, wobei in dem Leitungsbauteil (12; 14') eine Strömungskanalanordnung (62, 90; 70') ausgebildet ist, durch welche jeder Fluidleitungsabschnitt (bei 44, 96; 70a'-70d') der ersten, der zweiten und der dritten Ankopplungsformation (44, 18, 24; 30a'-30d') mit wenigstens einem, vorzugsweise mit jedem, Fluidleitungsabschnitt (bei 44, 96; 70a'-70d') einer, vorzugsweise jeder, der anderen beiden Ankopplungsformationen (44, 18, 24; 30a'-30d') zum Fluidtransport verbunden oder verbindbar ist.

2. Handhabungseinrichtung nach Anspruch 1,
**dadurch gekennzeichnet, dass** die Ventilleitungsanordnung (10') einen relativ zum Leitungsbauteil (14') beweglichen Fluidkanalträger (16') aufweist, welcher wenigstens zwei gesondert voneinander ausgebildete und mit unterschiedlichen Fluidkanälen zum Fluidtransport verbundene, jeweils von einem Fluidkanalabschnitt (20') durchsetzte Fluidkanalträger-Anschlussformationen (18', 19') aufweist, und wobei wenigstens eine der drei Ankopplungsformationen (30a'-30d') als Wechsel-Ankopplungsformation (30d') zur vorübergehenden Herstellung einer gemeinsamen Fluidleitungsstrecke mit einer durch Relativbewegung von Leitungsbauteil (14') und Fluidkanalträger (16') auswählbaren Fluidkanalträger-Anschlussformation (18', 19') ausgebildet ist.

3. Handhabungseinrichtung nach Anspruch 1 oder 2,
**dadurch gekennzeichnet, dass** eine Mehrzahl von Ankopplungsformationen (18, 24; 30b'-30d'), vorzugsweise alle Ankopplungsformationen (18, 24; 30a'-30d') mit Ausnahme einer Ankopplungsformation (44; 30a') zur vorübergehenden Ankopplung eines Fluidkanals eines Laborbehälters (22; 12), einen Ventilsitz (78) und einen Ventilkörper (74; 72') aufweist bzw. aufweisen, wobei der Ventilkörper (74; 72') in einem Betriebszustand mit für eine Fluiddurchleitung gesperrtem Fluidleitungsabschnitt (96; 70b'-70d') einer Ankopplungsformation (18, 24; 30b'-30d') auf dem Ventilsitz (78) ruht.

4. Handhabungseinrichtung nach einem der Ansprüche 1 bis 3,
**dadurch gekennzeichnet, dass** in der Strömungskanalanordnung (62, 90) abseits der Ankopplungsformationen (44, 18, 24) ein Ventilsitz (72) und ein Ventilkörper (74) zur strömungsmechanischen Trennung verschiedener Bereiche der Strömungskanalanordnung (62, 90) vorgesehen sind, wobei in einem Betriebszustand mit voneinander strömungsmechanisch getrennten Bereichen der Strömungskanalanordnung (62, 90) der Ventilkörper (74) auf dem Ventilsitz (72) ruht.

5. Handhabungseinrichtung nach Anspruch 3 oder 4,
**dadurch gekennzeichnet, dass** der Ventilkörper (74, 72') wenigstens teilweise, vorzugsweise vollständig, aus ferromagnetischem Material gebildet ist und dass der Ventilsitz (78) einen Magneten (104; 73b'-73d'), vorzugsweise einen ringförmigen Magneten (104, 73b'-73d'), am meisten bevorzugt einen den zugeordneten Fluidleitungsabschnitt (96; 70b'-70d') umgebenden, von Fluid durchströmbaren Magneten (104; 73b'-73d') aufweist, welcher den Ventilkörper (74; 72') magnetisch in eine Schließstellung vorspannt, in der der Ventilkörper (74; 72') auf dem Ventilsitz (78) ruht.

6. Handhabungseinrichtung nach einem der Ansprüche 1 bis 5,
**dadurch gekennzeichnet, dass** der Ventilkörper (74; 72') ferromagnetisches Material aufweist, insbesondere aus ferromagnetischem Material hergestellt ist, und dass die Ventilleitungsanordnung eine Schaltanordnung (14) mit lokal an den Ankopplungsformationen (44, 18, 24; 30b'-30d') veränderbarer magnetischer Feldstärke aufweist, mit welcher der Ventilkörper (74; 72') von dem Ventilsitz (78) lösbar ist.

7. Handhabungseinrichtung nach einem der Ansprüche 1 bis 6,
**dadurch gekennzeichnet, dass** sie ein Bewegungsteil (26; 13'), etwa einen Schlitten (26; 13') oder einen Wagen aufweist, welches zur vorübergehenden Aufnahme eines Laborbehälters (22; 12') ausgebildet ist und welches längs einer Anschlussbahn (A; B') an die Ventilleitungsanordnung (10') annäherbar und von dieser entfernbar ist.

8. Handhabungseinrichtung nach Anspruch 7,
**dadurch gekennzeichnet, dass** das Bewegungsteil (26) eine, vorzugsweise orthogonal zur Annäherungsbahn (A) orientierte, Stirnwand (30) aufweist, wobei in der Stirnwand (30) eine Ausnehmung (32) zur Aufnahme eines Abschnitts des Laborbehälters (22), vorzugsweise eines Halsabschnitts (34) eines Behälterhalses (36), vorgesehen ist.

9. Handhabungseinrichtung nach Anspruch 7 oder 8,
**dadurch gekennzeichnet, dass** das Bewegungsteil (26; 13') und die Ventilleitungsanordnung (10') wenigstens dann, wenn sich das Bewegungsteil (26; 13') in einer an die Ventilleitungsanordnung (10') angenäherten Stellung befindet, um eine gemeinsame, vorzugsweise orthogonal zur Anschlussbahn (A; B') verlaufende, Schwenkachse (S) schwenkbar an einem Tragrahmen (48) der Handhabungseinrichtung vorgesehen sind.

10. Handhabungseinrichtung nach Anspruch 9,
**dadurch gekennzeichnet, dass** als Schwenkantrieb ein elektromotorischer Antrieb (50) vorgesehen ist, welcher dazu eingerichtet ist, eine Antriebskraft auf eine mit dem Tragrahmen (48) zur gemeinsamen Schwenkbewegung gekoppelte Schwenkwelle (52) zu übertragen.

11. Handhabungseinrichtung nach Anspruch 10,
**dadurch gekennzeichnet, dass** der Schwenkantrieb (50) einen um eine mit der Schwenkwelle (52) drehfest verbundene Riemenscheibe (56) verlaufenden Riemen (54) oder/und ein Zahnradgetriebe umfasst.

12. Handhabungseinrichtung nach Anspruch 10,
**dadurch gekennzeichnet, dass** der Schwenkantrieb (50) unmittelbar mit der Schwenkwelle (52) gekoppelt ist oder die Schwenkwelle (52) ist.

## Claims

1. A handling device with a valve line arrangement (10'), in particular for a cell culture installation, for introducing a fluid into a laboratory vessel (22; 12'), in particular a cell culture container, and/or for discharging a fluid from the same, and with a supporting structure (10; 11') receiving the valve line arrangement, the valve line arrangement (10') comprising a line component (12; 14')
- with a first coupling formation (44; 30a') for the temporary coupling of a first fluid channel (32'), preferably of a fluid channel of a laboratory vessel (22; 12'),
- with a second coupling formation (18, 24; 30d') for the temporary or permanent coupling of a second fluid channel (20'), preferably of a fluid channel of a fluid reservoir, and
- with a third coupling formation (18, 24; 30b', 30c') for the temporary or permanent coupling of a third fluid channel (54', 56'), preferably of a discharge channel,
wherein each coupling formation (44, 18, 24' 30a'-30d') is penetrated by a fluid line section (at 44, 96; 70a'-70d'), wherein, in the line component (12' 14'), a flow channel arrangement (62, 90; 70') is formed, by means of which each fluid line section (at 44, 96; 70a'- 70d') of the first, of the second and of the third coupling formation (44, 18, 24; 30a'-30d') is or can be connected to at least one, preferably to each, fluid line section (at 44, 96; 70a'-70d') of one, preferably of each, of the other two coupling formations (44, 18, 24; 30a'-30d') for the fluid transport.

2. The handling device according to Claim 1,
**characterized in that** the valve line arrangement (10') comprises a fluid channel support (16') which is movable relative to the line component (14') and which comprises at least two fluid channel support connection formations (18', 19'), which are formed separately from one another and connected to different fluid channels for the fluid transport, and which are each penetrated by a fluid channel section (20'), and wherein at least one of the three coupling formations (30a'-30d') is designed as change coupling formation (30d') for the temporary establishment of a common fluid line section with a fluid channel support connection formation (18', 19') which can be selected by relative movement of line component (14') and fluid channel support (16').

3. The handling device according to Claim 1 or 2,
**characterized in that** a plurality of coupling formations (18, 24; 30b'-30d'), preferably all the coupling formations (18, 24; 30a'-30d'), except for a coupling formation (44; 30a') for the temporary coupling of a fluid channel of a laboratory vessel (22; 12), comprises or comprise a valve seat (78) and a valve body (74; 72'), wherein the valve body (74; 72'), in an operating state with a fluid line section (96; 70b'-70d') of a coupling formation (18, 24; 30b'-30d') being barred to fluid passage, rests on the valve seat (78).

4. The handling device according to any one of Claims 1 to 3,
**characterized in that**, in the flow channel arrangement (62, 90) away from the coupling formations (44, 18, 24), a valve seat (72) and a valve body (74) are provided for the fluid mechanical separation of different regions of the flow channel arrangement (62, 90), wherein, in an operating state with regions of the flow channel arrangement (62, 90) being fluidically separated from one another, the valve body (74) rests on the valve seat (72).

5. The handling device according to Claim 3 or 4,
**characterized in that** the valve body (74, 72') is formed at least partially, preferably completely, from ferromagnetic material, and **in that** the valve seat (78) comprises a magnet (104; 73b'-73d'), preferably an annular magnet (104, 73b'-73d'), most preferably a magnet (104, 73b'-73d') that surrounds the associated fluid line section (96; 70b'-70d') and through which fluid can flow, which prestresses the valve body (74; 72') magnetically into a closed position in which the valve body (74; 72') rests on the valve seat (78).

6. The handling device according to any one of Claims 1 to 5,
**characterized in that** the valve body (74; 72') comprises ferromagnetic material, in particular is made from ferromagnetic material, and **in that** the valve line arrangement comprises a switching arrangement (14) with a magnetic field strength that can be varied locally on the coupling formations (44, 18, 24; 30b'-30d'), by means of which the valve body (74, 72') can be detached from the valve seat (78).

7. The handling device according to any one of Claims 1 to 6,
**characterized in that** it comprises a moving part (26; 13'), for example, a carriage (26'; 13') or a cart, which is designed for the temporary accommodation of a laboratory vessel (22; 12') and which can be moved closer to or farther from the valve line arrangement (10') along a connection path (A; B').

8. The handling device according to Claim 7,
**characterized in that** the moving part (26) comprises an end wall (30), oriented preferably orthogonally to the approach path (A), wherein, in the end wall (30), a recess (32) is provided for receiving a section of the laboratory vessel (22), preferably a neck section (34) of a vessel neck (36).

9. The handling device according to Claim 7 or 8,
**characterized in that**, at least when the moving part (26; 13') is in a position moved closer to the valve line arrangement (10'), the moving part (26; 13') and the valve line arrangement (10') are provided on a support frame (48) of the handling device pivotable about a common pivot axis (S), which extends preferably orthogonally to the connection path (A; B').

10. The handling device according to Claim 9,
**characterized in that**, as pivot drive, an electric motor drive (50) is provided, which is set up to transmit a drive force to a pivot shaft (52) coupled to the support frame (48) for the common pivoting movement.

11. The handling device according to Claim 10,
**characterized in that** the pivot drive (50) comprises a belt (54) that runs around a pulley (56) connected in a rotationally fixed manner to the pivot shaft (52), and/or a gear drive.

12. The handling device according to Claim 10,
**characterized in that** the pivot drive (50) is coupled directly to the pivot shaft (52) or is the pivot shaft (52).

## Revendications

1. Dispositif de manipulation avec un arrangement de conduite de soupape (10'), en particulier pour un système de culture cellulaire, pour introduire un fluide dans un récipient de laboratoire (22 ; 12') en particulier un récipient de culture cellulaire, ou/et pour évacuer un fluide de celui-ci, et avec une structure de support (10 ; 11') recevant l'arrangement de conduite de soupape, l'arrangement de conduite de soupape (10') comprenant un composant de conduite (12 ; 14')
- avec une première formation d'accouplement (44 ; 30a') pour l'accouplement temporaire d'un premier canal de fluide (32'), de préférence d'un canal de fluide d'un récipient de laboratoire (22 ; 12'),
- avec une deuxième formation d'accouplement (18, 24 ; 30d') pour l'accouplement temporaire ou permanent d'un deuxième canal de fluide (20'), de préférence d'un canal de fluide d'un réservoir de fluide, et
- avec une troisième formation d'accouplement (18, 24 ; 30b', 30c') pour l'accouplement temporaire ou permanent d'un troisième canal de fluide (54', 56'), de préférence d'un canal d'évacuation,
où une section de conduite de fluide (à 44, 96 ; 70a'-70d') passe à travers les formations d'accouplement (44, 18, 24 ; 30a'-30d'), où un arrangement de canal d'écoulement (62, 90 ; 70') est formé dans le composant de conduite (12 ; 14'), par lequel chaque section de conduite de fluide (à 44, 96 ; 70a'-70d') des premières, deuxièmes et troisièmes formations d'accouplement (44, 18, 24 ; 30a'-30d') est liée ou peut être liée à au moins une, de préférence à chaque section de conduite de fluide (à 44, 96 ; 70a'-70d') d'une, de préférence de chacune des deux autres formations d'accouplement (44, 18, 24 ; 30a'-30d') pour le transport de fluide.

2. Dispositif de manipulation selon la revendication 1,
**caractérisé en ce que** l'arrangement de conduite de soupape (10') comprend un support de canal de fluide (16') qui est mobile par rapport au composant de conduite (14'), comprenant au moins deux formations de connexion de support de canal de fluide (18', 19') formées individuellement et connectées à des canaux de fluide différents pour le transport de fluide, par lesquelles passe une section de canal de fluide respective (20'), et où au moins une des trois formations d'accouplement (30a'-30d') est adaptée sous forme de formation d'accouplement interchangeable (30d') pour la réalisation temporaire d'une ligne commune de conduite de fluide avec une formation de connexion de support de canal de fluide (18', 19') sélectionnable par un mouvement relatif de composant de conduite (14') et de support de canal de fluide (16').

3. Dispositif de manipulation selon les revendications 1 ou 2,
**caractérisé en ce qu'**une pluralité de formations d'accouplement (18, 24 ; 30b'-30d'), de préférence toutes les formations d'accouplement (18, 24 ; 30a'-30d') à l'exception d'une formation d'accouplement (44, 30a') pour l'accouplement temporaire d'un canal de fluide d'un récipient de laboratoire (22 ; 12) comprend ou comprennent un siège de soupape (78) et un corps de soupape (74 ; 72'), où, dans un état opérationnel avec une section de conduite de fluide (96 ; 70b'-70d') bloquée au passage de fluide d'une formation d'accouplement (18, 24 ; 30b'-30d'), le corps de soupape (74 ; 72') repose sur le siège de soupape (78).

4. Dispositif de manipulation selon une des revendications 1 à 3,
**caractérisé en ce qu'**un siège de soupape (72) et un corps de soupape (74) sont prévus dans l'arrangement de canal d'écoulement (62, 90) loin des formations d'accouplement (44, 18, 24) pour la séparation du point de vue de la mécanique des fluides de différentes sections de l'arrangement de canal d'écoulement (62, 90), ou dans un état opérationnel avec des sections de l'arrangement de canal d'écoulement (62, 90) séparées du point de vue de la mécanique des fluides le corps de soupape (74) repose sur le siège de soupape (72).

5. Dispositif de manipulation selon la revendication 3 ou 4,
**caractérisé en ce que** le corps de soupape (74, 72') est réalisé au moins partiellement, de préférence entièrement de matériau ferromagnétique et **en ce que** le siège de soupape (78) comprend un aimant (104 ; 73b'-73d'), de préférence un aimant annulaire (104 ; 73b'-73d'), de manière la plus préférée un aimant entourant la section de conduite de fluide (96 ; 70b'-70d') associée par lequel du fluide peut passer et qui met en précontrainte le corps de soupape (74 ; 72') par aimantation dans une position d'arrêt où le corps de soupape (74 ; 72') repose sur le siège de soupape (78).

6. Dispositif de manipulation selon une des revendications 1 à 5,
**caractérisé en ce que** le corps de soupape (74 ; 72') comprend du matériau ferromagnétique, et est en particulier fait de matériau ferromagnétique, et **en ce que** l'arrangement de conduite de soupape comprend un arrangement de commutation (14) avec une intensité de champ localement réglable aux formations d'accouplement (44, 18, 24 ; 30b'-30d') par lequel le corps de soupape (74 ; 72') peut être détaché du siège de soupape (78).

7. Dispositif de manipulation selon une des revendications 1 à 6,
**caractérisé en ce qu'**il comprend une partie de mouvement (26 ; 13'), par exemple un coulisseau (26 ; 13') ou un chariot qui est adapté pour la réception temporaire d'un récipient de laboratoire (22 ; 12') et qui peut être approché ou éloigné de l'arrangement de conduite de soupape (10') le long d'une voie de connexion (A; B').

8. Dispositif de manipulation selon la revendication 7,
**caractérisé en ce que** la partie de mouvement (26) comprend une paroi frontale (30) orientée de préférence dans un sens orthogonal à la voie de rapprochement (A), une encoche (32) pour recevoir une section du récipient de laboratoire (22), de préférence d'une section col (34) d'un col de récipient (36) étant prévue dans la paroi frontale (30).

9. Dispositif de manipulation selon les revendications 7 ou 8,
**caractérisé en ce que** la partie de mouvement (26 ; 13') et l'arrangement de conduite de soupape (10') sont prévus à un cadre de support (48) du dispositif de manipulation de manière pivotable autour d'un axe de pivotement (S) commun qui est de préférence orthogonal à la voie de connexion (A, B'), au moins lorsque la partie de mouvement (26 ; 13') est dans une position rapprochée a l'arrangement de conduite de soupape (10').

10. Dispositif de manipulation selon la revendication 9,
**caractérisé en ce que** l'entrainement pivotant est prévu sous forme d'un entraînement électromoteur (50) qui est adapté pour transmettre une force d'entraînement sur un arbre de pivotement (52) accouplé au cadre de support (48) pour un mouvement pivotant commun.

11. Dispositif de manipulation selon la revendication 10,
**caractérisé en ce que** l'entrainement pivotant (50) comprend une courroie (54) passant autour d'une poulie (56) connectée pour une rotation avec l'arbre pivotant (52) ou/et une transmission par engrenage.

12. Dispositif de manipulation selon la revendication 10,
**caractérisé en ce que** l'entrainement pivotant (50) est accouplé directement à l'arbre pivotant (52) ou représente l'arbre pivotant (52).
